# EUROPEAN PATENT SPECIFICATION

(11) **EP 4 405 094 B1**
(45) Date of publication and mention of the grant of the patent: **18.12.2024**
(21) Application number: 23744343.7
(22) Date of filing: 28.06.2023
(51) Int. Cl.: B01J 19/00

(54) **HIGH DEFINITION MOLECULAR ARRAY FEATURE GENERATION USING PHOTORESIST**
ERZEUGUNG HOCHAUFLÖSENDER MOLEKULARER MATRIXMERKMALE UNTER VERWENDUNG VON FOTORESIST
GÉNÉRATION DE CARACTÉRISTIQUES DE RÉSEAU MOLÉCULAIRE À HAUTE DÉFINITION À L'AIDE D'UNE RÉSINE PHOTOSENSIBLE

(30) Priority: 29.06.2022 US 202263356928 P
(43) Date of publication of application: 31.07.2024
(60) Divisional application: 24211432.0
(73) Proprietor: 10X Genomics, Inc., Pleasanton, CA 94588-3260 (US)
(72) Inventor: PATTERSON, David, Michael, Pleasanton, California 94588-3260 (US); SHORT, Steven, William, Pleasanton, California 94588-3260 (US); WILK, Dieter, Pleasanton, California 94588-3260 (US); ZHUANG, Pingwei, Pleasanton, California 94588-3260 (US); ZIRALDO, Solongo Batjargal, Pleasanton, California 94588-3260 (US)
(74) Representative: J A Kemp LLP
(86) International application number: PCT/US2023/069222
(87) International publication number: WO 2024/006798

(56) References cited:
- WO-A1-2006/044956
- WO-A1-2020/159794
- WO-A2-00/33084
- DE-A1- 19 932 488

## Description

### FIELD

The present disclosure relates in some aspects to methods for manufacturing a molecular array using photoresist and the molecular array generated *in situ* on a substrate.

### BACKGROUND

Arrays of nucleic acids are an important tool in the biotechnology industry and related fields. There are two main ways of producing nucleic acid arrays in which the immobilized nucleic acids are covalently attached to the substrate surface, e.g., via in situ synthesis in which the nucleic acid polymer is grown on the surface of the substrate in a stepwise, nucleotide-by-nucleotide fashion, or via deposition of a full, pre-synthesized nucleic acid/polypeptide, cDNA fragment, etc., onto the surface of the array.

At present, there remains a need to generate molecular arrays for analyzing at high resolution (e.g., single cell scale resolution) the spatial expression patterns of large numbers of genes, proteins, or other biologically active molecules simultaneously. Provided are methods, uses and articles of manufacture that meet these needs.

WO 2006/044956 discloses methods for assembly of high-fidelity synthetic polynucleotides.

### SUMMARY

The invention provides a method for providing an array, comprising: a) irradiating a substrate comprising a plurality of masked spot regions and an unmasked boundary region between the plurality of masked spot regions, whereby a photoresist in the boundary region is degraded to render the boundary region available for blocking or ablation of oligonucleotide molecules in the boundary region, whereas the spot regions are protected by a photoresist in the spot regions from oligonucleotide blocking or ablation; b) blocking or ablating oligonucleotide molecules in the boundary region; c) irradiating the substrate to render an oligonucleotide molecule immobilized in a first one or more of the spot regions on the substrate available for oligonucleotide attachment, whereas an oligonucleotide molecule immobilized in one or more other spot regions on the substrate are not available for oligonucleotide attachment; and d) attaching a first oligonucleotide of at least four nucleotides in length to the oligonucleotide molecule immobilized in the first one or more of the spot regions to provide an extended oligonucleotide molecule in the first one or more of the spot regions. In some embodiments, the irradiating in step a) and blocking in step b) are performed before the irradiating in step c) and attaching in step d).

In any of the preceding embodiments, the method can comprise blocking oligonucleotide molecules in the boundary region by adding a 3' dideoxy, a non-ligating 3' phosphoramidate, or a triphenylmethyl (trityl) group to the oligonucleotide molecules in the boundary region. In some embodimnets, the method comprises blocking oligonucleotide molecules in the boundary region by adding a 3' dideoxy group to the oligonucleotide molecules in the boundary region. In some embodiments, the method comprises blocking oligonucleotide molecules in the boundary region by adding a 3' dideoxynucleotide to the oligonucleotide molecules in the boundary region. In any of the preceding embodiments, the addition can be catalyzed by a terminal transferase. In any of the preceding embodiments the terminal transferase is a terminal deoxynucleotidyl transferase (TdT).

In any of the preceding embodiments, the method can comprise ablating oligonucleotide molecules in the boundary region. In any of the preceding embodiments, ablating oligonucleotide molecules in the boundary region can comprise digesting the oligonucleotide molecules with an exonuclease. In some embodiments, the exonuclease is Exonuclease I. In any of the preceding embodiments, ablating oligonucleotide molecules in the boundary region can comprise performing plasma etching in the boundary region. In any of the preceding embodiments, the plasma etching can comprise exposing the boundary region to oxygen-based plasma. In any of the preceding embodiments, ablating oligonucleotide molecules may completely remove exposed oligonucleotide molecules in the boundary region.

In any of the preceding embodiments, prior to the irradiation in step c), the oligonucleotide molecules in the first one or more of the spot regions may be protected from hybridization and/or ligation. In any of the preceding embodiments, during and after the irradiation in step c), the oligonucleotide molecules in the one or more other spot regions may be protected from hybridization and/or ligation.

In any of the preceding embodiments, the oligonucleotide molecules in the spot region(s) may be protected from hybridization and/or ligation by a photoresist covering the oligonucleotide molecules. In any of the preceding embodiments, the photoresist in irradiated spot regions may be removed and the photoresist in masked or non-irradiated spot regions may not be removed.

In any of the preceding embodiments, the oligonucleotide molecules in the spot region(s) may be protected from hybridization and/or ligation by a protective group of each oligonucleotide molecule. In any of the preceding embodiments, the protective group can be a photo-cleavable protective group. In any of the preceding embodiments, the photo-cleavable protective groups in irradiated regions in step c) are cleaved and the photo-cleavable protective groups in masked or non-irradiated regions in step c) are not cleaved.

In any of the preceding embodiments, the oligonucleotide molecules in the spot region(s) may be protected from hybridization and/or ligation by a polymer binding to the oligonucleotide molecules. In any of the preceding embodiments, the polymer can be a photocleavable polymer. In some embodiments, the polymer binds to the oligonucleotide molecules in a non-sequence specific manner. In any of the preceding embodiments, the photo-cleavable polymers in the irradiated first one or more spot of the spot regions may be cleaved, while the photo-cleavable polymers in masked or non-irradiated one or more other spot regions are not cleaved.

In any of the preceding embodiments, the method can comprise irradiating the substrate in multiple cycles, each cycle for irradiating one or more spot regions that are different from the one or more spot region(s) irradiated in another cycle and attaching an oligonucleotide of at least four nucleotides in length to the oligonucleotide molecule immobilized in irradiated spot region(s) for the cycle. In any of the preceding embodiments, the irradiating in step c) can be performed using a photomask wherein the first one or more of the spot regions are unmasked and the one or more other spot regions are masked. In any of the preceding embodiments, the method can comprise translating the photomask used in the irradiating step c) from a first position to a second position relative to the substrate, each position for a cycle of irradiating the substrate and attaching an oligonucleotide.

In any of the preceding embodiments, oligonucleotide molecules on the substrate comprise one or more common sequences. In some embodiments, the one or more common sequences comprise a common primer. In some embodiments, the common sequence is between about 10 and about 35 nucleotides in length.

In any of the preceding embodiments, oligonucleotide molecules in the first one or more of the spot regions, the one or more other spot regions, and the boundary region can be identical in sequence prior to the irradiating in step a).

In any of the preceding embodiments, the plurality of spot regions may be, on average, no more than 55 micrometers in diameter. In some embodiments, the plurality of spot regions are each no more than 55 micrometers in diameter. In any of the preceding embodiments, the plurality of spot regions may be, on average, no more than 5 micrometers in diameter. In some embodiments, the plurality of spot regions are each no more than 5 micrometers in diameter.

In any of the preceding embodiments, the boundary region can comprise the region on the substrate surrounding and between the plurality of spot regions. In any of the preceding embodiments, oligonucleotide molecules on the substrate prior to the irradiating step may be between about 4 and about 50 nucleotides in length. In any of the preceding embodiments, the oligonucleotide molecules may be applied to the substrate as a lawn prior to step a).

### BRIEF DESCRIPTION OF THE DRAWINGS

The drawings illustrate certain features and advantages of this disclosure. These embodiments are not intended to limit the scope of the appended claims in any manner.
**FIG. 1** shows an exemplary method for *in situ* array generation using photoresist, wherein the photoresist is degraded in a boundary region to render oligonucleotide molecules in the boundary region available for oligonucleotide blocking or ablation. After blocking or ablating oligonucleotide molecules in the boundary region, the photoresist is removed and oligonucleotides previously covered with photoresist are available for ligation in a spot region surrounded by the boundary region.
**FIG. 2** is a schematic illustration of an exemplary method for array generation comprising photoresist degradation and oligonucleotide blocking in a boundary region surrounding a plurality of spot regions.
**FIG. 3** shows an exemplary photomask for selectively irradiating a boundary region around a plurality of spot regions, wherein the spot regions are protected by the photomask.
**FIG. 4** shows an exemplary method for *in situ* array generation using photoresist, wherein the photoresist is degraded in a boundary region to render oligonucleotide molecules in the boundary region available for oligonucleotide blocking or digestion. After blocking or digesting oligonucleotide molecules in the boundary region, the photoresist is removed and oligonucleotides previously covered with photoresist are available for ligation in a spot region surrounded by the boundary region.
**FIG. 5** shows an image of two arrays generated either via 3' capping or Exonuclease I digestion of the boundary between features prior to photo-hybridization/ligation reactions.

### DETAILED DESCRIPTION

The technical information set out below may in some respects go beyond the disclosure of the invention, which is defined by the appended claims. The additional technical information is provided to place the invention in a broader technical context and to illustrate possible related technical developments. Such additional technical information which does not fall within the scope of the appended claims is not part of the invention. Accordingly, any aspects, embodiments and examples of the present disclosure which do not fall under the scope of the appended claims do not form part of the invention and are merely provided for illustrative purposes.

The practice of the techniques described herein may employ, unless otherwise indicated, conventional techniques and descriptions of organic chemistry, polymer technology, molecular biology (comprising recombinant techniques), cell biology, biochemistry, and sequencing technology, which are within the skill of those who practice in the art. Such conventional techniques comprise polymer array synthesis, hybridization and ligation of polynucleotides, and detection of hybridization using a label. Specific illustrations of suitable techniques can be had by reference to the examples herein. However, other equivalent conventional procedures can, of course, also be used. Such conventional techniques and descriptions can be found in standard laboratory manuals such as Green, et al., Eds. (1999), Genome Analysis: A Laboratory Manual Series (Vols. I-IV); Weiner, Gabriel, Stephens, Eds. (2007), Genetic Variation: A Laboratory Manual; Dieffenbach, Dveksler, Eds. (2003), PCR Primer: A Laboratory Manual; Bowtell and Sambrook (2003), DNAMicroarrays: A Molecular Cloning Manual; Mount (2004), Bioinformatics: Sequence and Genome Analysis; Sambrook and Russell (2006), Condensed Protocols from Molecular Cloning: A Laboratory Manual; and Sambrook and Russell (2002), Molecular Cloning: A Laboratory Manual (all from Cold Spring Harbor Laboratory Press); Stryer, L. (1995) Biochemistry (4th Ed.) W. H. Freeman, New York N.Y.; Gait, "Oligonucleotide Synthesis: A Practical Approach" 1984, IRL Press, London; Nelson and Cox (2000), Lehninger, Principles of Biochemistry 3rd Ed., W. H. Freeman Pub., New York, N.Y.; and Berg et al. (2002) Biochemistry, 5th Ed., W. H. Freeman Pub., New York, N.Y..

If a definition set forth herein is contrary to or otherwise inconsistent with a definition set forth in the patents, applications, published applications and other publications that are referenced, the definition set forth herein prevails over the definition that is in the reference.

The section headings used herein are for organizational purposes only and are not to be construed as limiting the subject matter described.

### I. OVERVIEW

There is a need for oligonucleotide arrays providing barcoded oligonucleotide molecules at known positions with high purity and fidelity for various applications, including but not limited to high-resolution spatial transcriptomic analysis. However, methods to decrease spot sizes or features at or below 10 microns (e.g., single cell scale resolution) in diameter with sufficient throughput are lacking. In some aspects, provided herein are methods for the fabrication of patterned arrays (e.g., a substrate having coupled to it a plurality of polymer molecules, such as oligonucleotides) with high spatial resolution using a photoresist, comprising degrading a photoresist in a boundary region surrounding spots or features of an array and blocking or ablating oligonucleotides in the boundary region to prevent attachment of nucleotides or ligation of oligonucleotides in the boundary region, whereas the oligonucleotides in the spots or features are protected from blocking or ablation by the photoresist. Provided herein in some embodiments are methods and uses of light-controlled combinatorial barcode generation for *in situ* arrays (e.g., after blocking or ablating oligonucleotide molecules in the boundary region). In some embodiments, light-controlled ligation for *in situ* combinatorial barcode generation is utilized.

Methods for in situ generated arrays have utilized photo-cleavable protecting groups to synthesize barcode oligonucleotides one nucleotide at a time. The feature size is controlled using photomasks to cover boundary regions between spots and features, whereas photoresist covering features (e.g., spots) is degraded. However, the size and resolution of individual features is limited by the ability to specifically degrade photoresist in the features and block erroneous nucleotide incorporation in the boundary regions using photoresist.

In some aspects, provided herein is a method of generating an array comprising a step of degrading photoresist in the boundary region between features, whereas the features (e.g., spots) are protected by a photoresist. After degrading the photoresist in the boundary region, oligonucleotide molecules in the boundary region are blocked or ablated (e.g., digested by using a nuclease or removed using plasma etching). In some embodiments, the method comprises a single step of blocking or ablating oligonucleotide molecules in the boundary region using a single photomask designed to expose the boundary regions to irradiation while protecting the features, followed by one or more cycles of oligonucleotide ligation with exposed features (optionally wherein selected features are exposed using a photomask in each cycle). As described herein, the immobilized nucleic acid generated using a method provided herein can provide a higher resolution and/or higher barcode accuracy compared to methods of light mediated base-by-base in situ synthesis without blocking/degrading oligonucleotide molecules in the boundary region.

In some aspects, provided herein is a method for providing an array, comprising: a) irradiating a substrate comprising a plurality of masked spot regions and an unmasked boundary region between the plurality of masked spot regions, whereby a photoresist in the boundary region is degraded to render the boundary region available for blocking or ablation of oligonucleotide molecules in the boundary region, whereas the spot regions are protected by a photoresist in the spot regions from oligonucleotide blocking or ablation; b) blocking or ablating oligonucleotide molecules in the boundary region; c) irradiating the substrate to render an oligonucleotide molecule immobilized in a first one or more of the spot regions on the substrate available for oligonucleotide attachment, whereas an oligonucleotide molecule immobilized in one or more other spot regions on the substrate are not available for oligonucleotide attachment; and d) attaching a first oligonucleotide of at least four nucleotides in length to the oligonucleotide molecule immobilized in the first one or more of the spot regions to provide an extended oligonucleotide molecule in the first one or more of the spot regions.

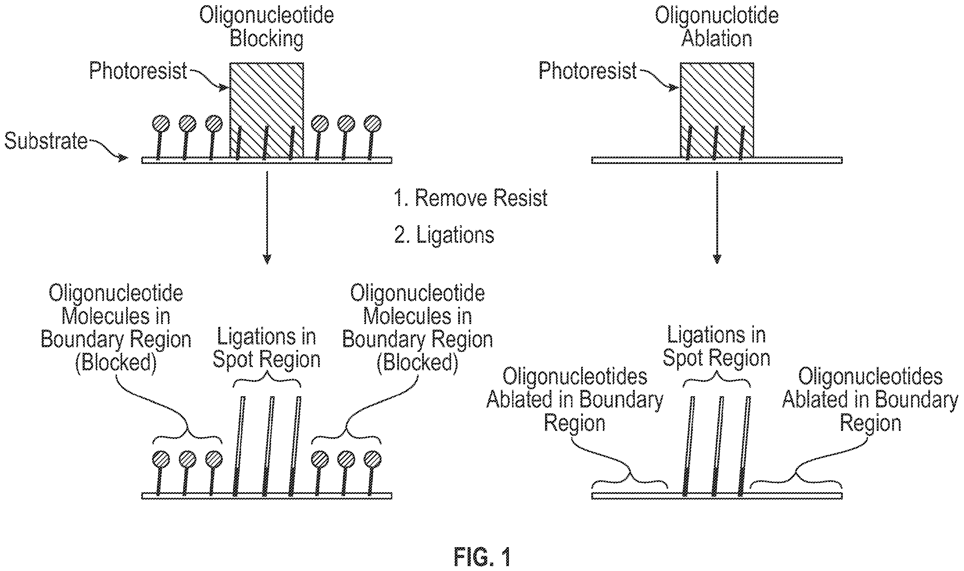

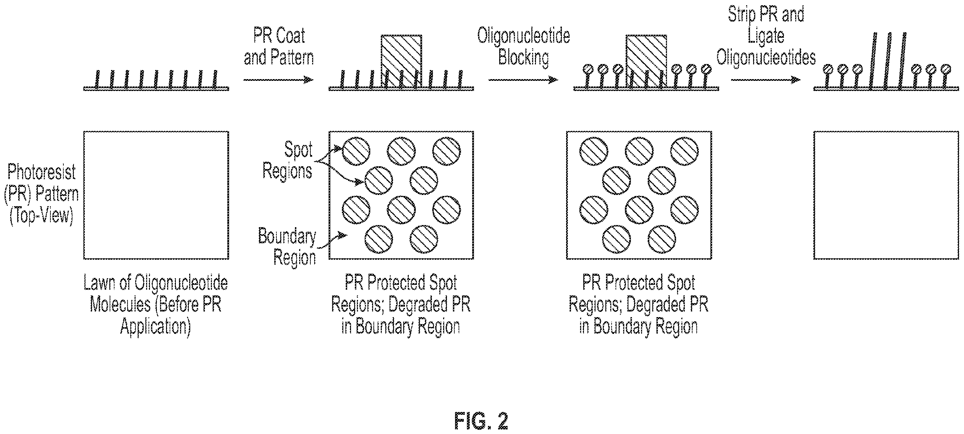

In some embodiments, the method comprises blocking oligonucleotide molecules in the boundary region after irradiating the substrate to degrade a photoresist in the boundary region but not in the spot regions (e.g., as shown in **FIG. 1**). In some embodiments, the method comprises blocking oligonucleotide molecules in the boundary region by adding a 3' dideoxy, a non-ligating 3' phosphoramidate, or a triphenylmethyl (trityl) group to the oligonucleotide molecules in the boundary region. In some embodiments, the method comprises blocking oligonucleotide molecules in the boundary region by adding a 3' dideoxy group to the oligonucleotide molecules in the boundary region. In some embodiments, the addition is catalyzed by a terminal transferase. In some embodiments, the terminal transferase is a terminal deoxynucleotidyl transferase (TdT). **FIG. 2** shows an additional example of a method comprising blocking oligonucleotide molecules in the boundary region, wherein the substrate is irradiated to degrade a photoresist in the boundary region while oligonucleotide molecules immobilized in spot regions on the substrate are protected from blocking, followed by removal of the photoresist and ligation of oligonucleotides to oligonucleotide molecules in the spot regions. The bottom panel of **FIG. 2** provides a top view showing photoresist protected spots (shaded spots) and the boundary region comprising degraded photoresist (white region) in an exemplary method.

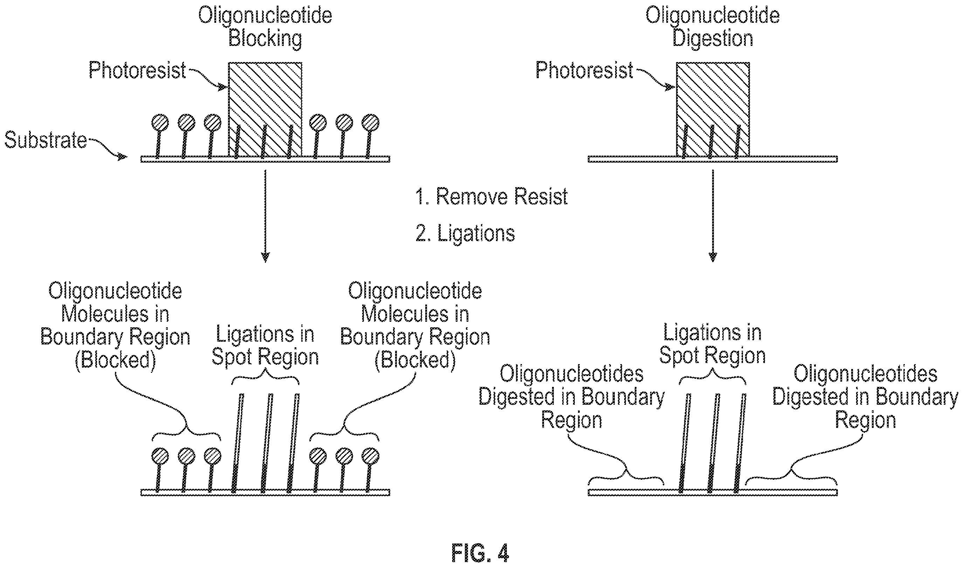

In some embodiments, the method comprises ablating oligonucleotide molecules in the boundary region after irradiating the substrate to degrade a photoresist in the boundary region but not in the spot regions (e.g., as shown in **FIG. 1**). In some embodiments, ablating oligonucleotide molecules in the boundary region comprises digesting the oligonucleotide molecules with an exonuclease (e.g., as shown in **FIG. 4**). In some embodiments, ablating oligonucleotide molecules in the boundary region comprises performing plasma etching in the boundary region. In some embodimnets, the plasma etching comprises exposing the boundary region to oxygen-based plasma. In some embodiments, ablating oligonucleotide molecules in the boundary region is performed before ligation of oligonucleotides in the spot regions. In other embodiments, ablating oligonucleotide molecules in the boundary region is performed after ligation of oligonucleotides in the spot regions (e.g., after one or more rounds of ligation in the same spot region and/or after one or more cycles of ligation in different spot regions.

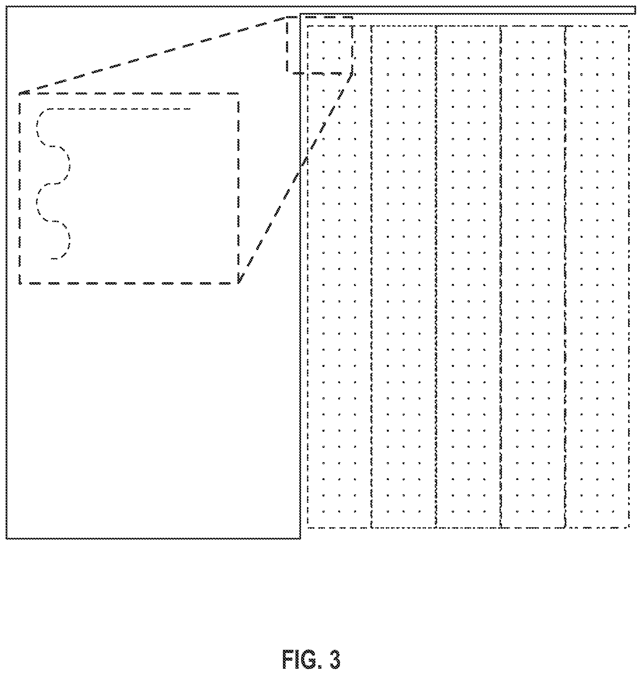

In some embodiments, irradiating a substrate comprising a plurality of masked spot regions and an unmasked boundary region between the plurality of masked spot regions comprises irradiating the substrate through a photomask comprising masked "spots" or features. An exemplary photomask is shown in **FIG. 3****,** comprising masked spots and open boundary regions between the spots. In some embodiments, the spots are 4x5 µM circles (e.g., the spots can be elongated circles). In some embodiments, one or more edges of the photomask are sinusoidal (e.g., as shown in **FIG. 3**), allowing translation of the photomask to create densely packed spots.

In some aspects, before or after blocking or ablating oligonucleotide molecules in the boundary region, the methods provided herein comprise photohybridization-ligation combinatorial barcode generation using a photoresist and photolithography for *in situ* arrays. For example, a method disclosed herein may comprise photocontrollable ligation, wherein localized irradiation causes degradation of photoresist and oligonucleotides to be exposed for ligation. In some aspects, a method disclosed herein provides one or more advantages as compared to available arraying methods. For example, a large diversity of barcodes can be created via sequential rounds of hybridization and ligation. In some embodiments, no protection/deprotection step is required for ligating oligonucleotides to the substrate; the feature size can be highly controlled (e.g., submicron scale) using the blocking or ablation of oligonucleotide molecules in the boundary region using photomasks, and the generated array at any discrete location is known and consistent (e.g., no nucleotide incorporation errors) across all arrays with no decoding needed. In some embodiments, the blocking or ablating step reduces erroneous barcoding of oligonucleotide molecules in the boundary region.

In some embodiments, the method described herein comprises a plurality of rounds of ligation wherein each round comprises one or more cycles. In some embodiments, each cycle within the same round comprises the following general steps: (1) selective removal of a photoresist, photocleavable moiety, or photocleavable polymer by irradiation/UV exposure; (2) ligation of an oligonucleotide; (3) blocking or capping, wherein the steps are reiterated for different features (also referred to herein as "spots"). In some embodiments, each feature receives at most one oligonucleotide in a round, wherein all features are ligated to at most one part of one or more barcodes.

### II. PHOTOLITHOGRAPHY AND FEATURE PATTERNING BY BLOCKING OR ABLATING OLIGONUCLEOTIDES IN BOUNDARY REGIONS

A photoresist is a light-sensitive material used in processes (such as photolithography and photoengraving) to form a pattern on a surface. A photoresist may comprise a polymer, a sensitizer, and/or a solvent. The photoresist composition used herein is not limited to any specific proportions of the various components.

Photoresists can be classified as positive or negative. In positive photoresists, the photochemical reaction that occurs during light exposure weakens the polymer, making it more soluble to developer, so a positive pattern is achieved. In the case of negative photoresists, exposure to light causes polymerization of the photoresist, and therefore the negative photoresist remains on the surface of the substrate where it is exposed, and the developer solution removes only the unexposed areas. In some embodiments, the photoresist used herein is a positive photoresist. In some embodiments, the photoresist is degraded or removable with UV light.

In some aspects, disclosed herein is a method for providing an array, comprising: (a) irradiating a substrate comprising an unmasked first region and a masked second region, whereby a photoresist in the first region is degraded to render oligonucleotide molecules in the first region available for blocking and/or exonuclease digestion whereas oligonucleotide molecules in the second region are protected from blocking and/or exonuclease digestion, and (b) blocking or digesting oligonucleotide molecules in the first region; (c) rendering oligonucleotide molecules in the second region available for attachment of nucleotides or oligonucleotides and contacting the substrate with a first nucleotide or an oligonucleotide at least 4 nucleotides in length, and attaching the first nucleotide or oligonucleotide to the oligonucleotide molecules in the second region. In some embodiments, the first and second region are coated with a photoresist prior to step (a). In some embodiments, the first region is a boundary region, and the second region is one or more features (e.g., spots), wherein barcoded oligonucleotide molecules are generated in the features.

In some embodiments, the feature(s) (e.g., spots) are no more than 5 microns, no more than 10 microns, no more than 15 microns, no more than 20 microns, no more than 25 microns, no more than 30 microns, no more than 35 microns, no more than 40 microns, no more than 45 microns, no more than 50 microns, or no more than 55 microns in diameter. In some embodiments, the feature(s) (e.g., spots) are no more than 5 microns in diameter. In some embodiments, the feature(s) (e.g., spots) are no more than 55 microns in diameter. In some embodiments, the feature(s) (e.g., spots) are about 4 to about 10 microns in diameter. In some embodiments, the plurality of spot regions are, on average, no more than 55 micrometers in diameter. In some embodiments, the plurality of spot regions are, on average, no more than 5 micrometers in diameter. In some embodiments, the plurality of spot regions are each no more than 55 micrometers in diameter. In some embodiments, the plurality of spot regions are each no more than 5 micrometers in diameter.

In some embodiments, the method comprises blocking oligonucleotide molecules in the boundary region after irradiating the substrate to degrade a photoresist in the boundary region but not in the spot regions (e.g., as shown in **FIG. 1**). The boundary region comprises the region on the substrate surrounding and between the plurality of spot regions. In some embodiments, the method comprises blocking oligonucleotide molecules in the boundary region by adding a 3' dideoxy, a non-ligating 3' phosphoramidate, or a triphenylmethyl (trityl) group to the oligonucleotide molecules in the boundary region. In some embodiments, the method comprises blocking oligonucleotide molecules in the boundary region by adding a 3' dideoxy group to the oligonucleotide molecules in the boundary region. In some embodiments, the addition is catalyzed by a terminal transferase. In some embodiments, the terminal transferase is a terminal deoxynucleotidyl transferase (TdT). In some embodiments, the method comprises contacting the substrate (e.g., contacting the exposed boundary region of the substrate) with a TdT and a nucleotide comprising a blocking group (such as a 3' dideoxynucleotide, e.g., ddTTP, ddATP, ddCTP, ddGTP). In some embodiments, the method comprises incubating the substrate with the TdT and the dideoxynucleotide (ddNTP) for at least 5 minutes, at least 10 minutes, at least 15 minutes, at least 20 minutes, or at least 30 minutes. In some embodiments, the method comprises incubating the substrate with the TdT and the dideoxynucleotide (ddNTP) for between about 5 and about 10 minutes, between about 5 and about 20 minutes, between about 5 and about 30 minutes, between about 10 and about 15 minutes, between about 10 and about 30 minutes, or between about 20 and about 30 minutes. In some embodiments, the method comprises incubating the substrate with the TdT and the dideoxynucleotide (ddNTP) for no more than about 30 minutes or about 40 minutes. In some embodiments, the substrate is incubated with the TdT and the ddNTP at room temperature (e.g., about 20 °C or about 22 °C). In some embodiments, the substrate is incubated with the TdT and the ddNTP at about 37 °C.

In some embodiments, the method comprises ablating oligonucleotide molecules in the boundary region after irradiating the substrate to degrade a photoresist in the boundary region but not in the spot regions (e.g., as shown in **FIG. 1**). In some embodiments, ablating oligonucleotide molecules in the boundary region comprises digesting the oligonucleotide molecules with an exonuclease. In some embodiments, the exonuclease is a 3' exonuclease. In some embodiments, the exonuclease is Exonuclease I. In some embodiments, ablating oligonucleotide molecules in the boundary region comprises performing plasma etching in the boundary region. In some embodiments, the plasma etching comprises exposing the boundary region to oxygen-based plasma. In some embodiments, ablating oligonucleotide molecules in the boundary region is performed before ligation of oligonucleotides in the spot regions. In other embodiments, ablating oligonucleotide molecules in the boundary region is performed after ligation of oligonucleotides in the spot regions (e.g., after one or more rounds of ligation in the same spot region and/or after one or more cycles of ligation in different spot regions).

The photoresist may experience changes in pH upon irradiation. In some embodiments, the photoresist in the first region comprises a photoacid generator (PAG). In some embodiments, the photoresist in the second region comprises a photoacid generator. In some embodiments, the photoresist in the first and the second region comprises a photoacid generator. In some embodiments, the photoresist in the first and the second region comprises the same photoacid generator. In some embodiments, the photoresist in the first and the second region comprises different photoacid generators. In some embodiments, the photoacid generator or photoacid generators irreversibly release protons upon absorption of light. Photoacid generators may be used as components of photocurable polymer formulations and chemically amplified photoresists. Examples of photoacid generators include triphenylsulfonium triflate, diphenylsulfonium triflate, diphenyliodonium nitrate, N-Hydroxynaphthalimide triflate, triarylsulfonium hexafluorophosphate salts, N-hydroxy-5-norbornene-2,3-dicarboximide perfluoro-1-butanesulfonate, bis(4-tert-butylphenyl)iodonium perfluoro-1-butanesulfonate, triphenylsulfonium trifluoromethanesulfonate, triphenylsulfonium nonafluoro-n-butanesulfonate, triphenylsulfonium perfluoro-n-octanesulfonate, and triphenylsulfonium 2-(bicyclo[2.2. 1]heptan-2-yl)-1, 1,2,2-tetrafluoroethanesulfonate, 4-cyclohexylphenyldiphenylsulfonium trifluoromethanesulfonate, 4-cyclohexylphenyldiphenylsulfonium nonafluoro-n-butanesulfonate, 4-cyclohexylphenyldiphenylsulfonium perfluoro-n-octanesulfonate, 4-cyclohexylphenyldiphenylsulfonium 2-(bicyclo[2.2.1]heptan-2-yl)-1,1,2,2-tetrafluoroethanesulfonate, 4-methanesulfonylphenyldiphenylsulfonium trifluoromethanesulfonate, 4-methanesulfonylphenyldiphenylsulfonium nonafluoro-n-butanesulfonate, 4-methanesulfonylphenyldiphenylsulfonium perfluoro-n-octanesulfonate, and 4-methanesulfonylphenyldiphenylsulfonium 2-(bicyclo[2.2. 1]heptan-2-yl)-1, 1,2,2-tetrafluoroethanesulfonate, diphenyliodonium trifluoromethanesulfonate, diphenyliodonium nonafluoro-n-butanesulfonate, diphenyliodonium perfluoro-n-octanesulfonate, diphenyliodonium 2-(bicyclo[2.2. 1]heptan-2-yl)-1, 1,2,2-tetrafluoroethanesulfonate, bis(4-t-butylphenyl)iodonium trifluoromethanesulfonate, bis(4-t-butylphenyl)iodonium nonafluoro-n-butanesulfonate, bis(4-t-butylphenyl)iodonium perfluoro-n-octanesulfonate, bis(4-t-butylphenyl)iodonium 2-(bicyclo[2.2.1]heptan-2-yl)-1,1,2,2-tetrafluoroethanesulfonate, 1-(4-n-butoxynaphthalen-1-yl)tetrahydrothiophenium trifluoromethanesulfonate, 1-(4-n-butoxynaphthalen-1-yl)tetrahydrothiophenium nonafluoro-n-butanesulfonate, 1-(4-n-butoxynaphthalen-1-yl)tetrahydrothiophenium perfluoro-n-octanesulfonate, 1-(4-n-butoxynaphthalen-1-yl)tetrahydrothiophenium 2-(bicyclo[2.2.1]heptan-2-yl)-1,1,2,2-tetrafluoroethanesulfonate, 1-(6-n-butoxynaphthalen-2-yl)tetrahydrothiophenium trifluoromethanesulfonate, 1-(6-n-butoxynaphthalen-2-yl)tetrahydrothiophenium nonafluoro-n-butanesulfonate, 1-(6-n-butoxynaphthalen-2-yl)tetrahydrothiophenium perfluoro-n-octanesulfonate, 1-(6-n-butoxynaphthalen-2-yl)tetrahydrothiophenium 2-(bicyclo[2.2.1]heptan-2-yl)-1,1,2,2-tetrafluoroethanesulfonate, 1-(3,5-dimethyl-4-hydroxyphenyl)tetrahydrothiophenium trifluoromethanesulfonate, 1-(3,5-dimethyl-4-hydroxyphenyl)tetrahydrothiophenium nonafluoro-n-butanesulfonate, 1-(3,5-dimethyl-4-hydroxyphenyl)tetrahydrothiophenium perfluoro-n-octanesulfonate, 1-(3,5-dimethyl-4-hydroxyphenyl)tetrahydrothiophenium 2-(bicyclo[2.2.1]heptan-2-yl)-1,1,2,2-tetrafluoroethanesulfonate N-(trifluoromethanesulfonyloxy)bicyclo[2.2.1]hept-5-ene-2,3-dicarboximide, N-(nonafluoro-n-butanesulfonyloxy)bicyclo[2.2. 1]hept-5-ene-2,3-dicarboxylmide, N-(perfluoro-n-octanesulfonyloxy)bicyclo[2.2.1]hept-5-ene-2,3-dicarboxylmide, N-[2-(bicyclo[2.2.1]heptan-2-yl)-1,1,2,2-tetrafluoroethanesulfonyloxy]bicyclo[2.2.1]hept-5-ene-2,3-dicarboxylmide, N-[2-(tetracyclo[4.4.0.12,5.17,10]dodecan-3-yl)-1,1-difluoroethanesulfonyloxy]bicyclo[2.2.1]hept-5-ene-2,3-dicarboxylmide, 1,3-dioxoisoindolin-2-yl trifluoromethanesulfonate, 1,3-dioxoisoindolin-2-yl nonafluoro-n-butane sulfonate, 1,3-dioxoisoindolin-2-yl perfluoro-n-octane sulfonate, 3-dioxoisoindolin-2-yl 2-(bicyclo[2.2.1]heptan-2-yl)-1,1,2,2-tetrafluoroethanesulfonate, 3-dioxoisoindolin-2-yl N42-(tetracyclo[4.4.0.12,5.17,10]dodecan-3-yl)-1,1-difluoroethanesulfonate, 1,3-dioxo-1H-benzo[de]isoquinolin-2(3H)-yl trifluoromethanesulfonate, 1,3-dioxo-1H-benzo[de]isoquinolin-2(3H)-yl nonafluoro-n-butane sulfonate, 1,3-dioxo-1H-benzo[de]isoquinolin-2(3H)-yl perfluoro-n-octanesulfonate, 1,3-dioxo-1H-benzo[de]isoquinolin-2(3H)-yl 2-(bicyclo[2.2.1]heptan-2-yl)-1,1,2,2-tetrafluoroethanesulfonate, or 1,3-dioxo-1H-benzo[de]isoquinolin-2(3H)-yl N-[2-(tetracyclo[4.4.0.12,5.17,10]dodecan-3-yl)-1,1-difluoroethanesulfonate, (E)-2-(4-methoxystyryl)-4,6-bis(trichloromethyl)-1,3,5-triazine, 2-(Methoxyphenyl)-4,6-bis-(trichloromethyl)-s-triazine, 2-[2-(Furan-2-yl)ethenyl]-4,6-bis(trichloromethyl)-s-triazine, 2-[2-(5-methylfuran-2-yl]ethenyl)-4,6-bis(trichloromethyl)-s-triazine, 2-[2-(3,4-Dimethoxyphenyl)ethenyl]-4,6-bis(trichloromethyl)-s-triazine, equivalents thereof or combinations thereof. In some cases, photoacid generators capable of generating perfluoroalkanesulfonic acid having a high acid strength are used as the PAG in the formulations of the present disclosure. Such photoacid generators include, but are not limited to, photoacid generators capable of generating partially fluorinated alkane sulfonic acids, fully fluorinated alkane sulfonic acids, perfluorohexanesulfonic acid, perfluorooctanesulfonic acid, perfluoro-4-ethylcyclohexanesulfonic acid, perfluoroalkyl ether sulfonic acids, and perfluorobutanesulfonic acid. Additional examples of photoacid generators are described in U.S. Patent Pub. No. 20200384436 and U.S. Patent Pub. No. 20210017127.

In some embodiments, a photoresist composition can form a micro or nanopattern used in lithography process for manufacturing a biomolecular array. In some embodiments, the lithography process uses a substrate material such as a wafer, e.g., a silicon-based wafer. In some embodiments, a thin photoresist layer is formed on a substrate, and then the substrate is optionally baked to fix the photoresist layer on the substrate. In some embodiments, the photoresist layer on the substrate is exposed to radiation. The exposed photoresist layer can be treated with a developing solution, and by dissolving and removing the exposed area of the photoresist layer, a micro or nanopattern is formed. In some embodiments, a photolithography process disclosed herein may comprise forming a photoresist layer on a substrate using a photoresist composition; selectively exposing the photoresist layer; and developing the exposed photoresist layer. In some embodiments, a photolithography process disclosed herein comprises coating a photoresist composition on a substrate and drying (soft baking) the coated substrate. In some embodiments, a photolithography process disclosed herein comprises coating with a spin coater, a bar coater, a blade coater, a curtain coater, a screen printer or the like, and/or a spray coater or the like, and any method capable of coating a photoresist composition may be used. Drying (soft baking) of the substrate may be preformed under a suitable condition and may comprise, for example, an oven, a hot plate, vacuum drying and the like, but is not limited thereto. When going through the drying, a solvent is removed from the photoresist composition, increasing adhesive strength between the wafer and the photosensitive resin layer, and the photoresist layer may be secured on the substrate. In some embodiments, the selectively exposing of the photoresist layer is performed by aligning a mask on the photoresist, and exposing an area of the photoresist layer not covered by the mask to ultraviolet rays. The mask may be in contact with the photoresist layer, or may also be aligned at a certain distance from the photoresist layer. In some embodiments, a light source irradiated as a light irradiation means may comprise electromagnetic waves, extreme ultraviolet rays (EUV), from ultraviolet rays to visible rays, an electron beam, X-rays, laser rays and the like. Known means such as a high pressure mercury lamp, a xenon lamp, a carbon arc lamp, a halogen lamp, a cold cathode tube for a copier, an LED and a semiconductor laser may be used. In some embodiments, the selectively exposing of the photoresist layer may further comprise heating (post-exposure baking) the exposed photoresist layer after the exposure. In some embodiments, developing of the exposed photoresist layer comprises removing the exposed portion in the photoresist layer by immersing in a developing solution. Any photoresist developing methods known in the art may be used and are not limited to a rotary spray method, a paddle method, or an immersion method accompanying ultrasonic treatment. Examples of the developing solution may comprise alkali metal or alkaline earth metal hydroxides, carbonates, hydrogen carbonates, an aqueous basic solution such as an ammonia water quaternary ammonium salt may be used. For instance, an aqueous ammonia quaternary ammonium solution such as an aqueous tetramethyl ammonium solution may be used.

In some embodiments, the photoresist further comprises an acid scavenger. In some embodiments, the photoresist in the first and the second region comprises the same acid scavenger. In some embodiments, the photoresist in the first and the second region comprises different acid scavengers. In some embodiments, an acid scavenger acts to neutralize, adsorb and/or buffer acids, and may comprise a base or alkaline compound. In some embodiments, acid scavengers act to reduce the amount or concentration of protons or protonated water. In some embodiments, an acid scavenger acts to neutralize, diminish, or buffer acid produced by a photoacid generator. In some embodiments, an acid scavenger exhibits little or no stratification over time or following exposure to heat. In some embodiments, acid scavengers may be further subdivided into "organic bases" and "polymeric bases." A polymeric base is an acid scavenger (e.g., basic unit) attached to a longer polymeric unit. A polymer is typically composed of a number of coupled or linked monomers. The monomers can be the same (to form a homopolymer) or different (to form a copolymer). In a polymeric base, at least some of the monomers act as acid scavengers. An organic base is a base which is joined to or part of a non-polymeric unit. Non-limiting examples of organic bases include, without limitation, amine compounds (e.g., primary, secondary and tertiary amines). Generally any type of acid scavenger, defined here as a traditional Lewis Base, an electron pair donor, can be used in accordance with the present disclosure. The acid scavenger may be a tertiary aliphatic amine or a hindered amine. Examples of the acid scavenger include, but are not limited to 2,2,6,6-tetramethyl-4- piperidyl stearate, l,2,2,6,6-pentamethyl-4-piperidyl stearate, 2,2,6,6-tetramethyl-4-piperidyl benzoate, bis(2,2,6,6-tetramethyl-4-piperidyl) sebacate, bis(l,2,2,6,6-tetramethyl-4-piperidyl) sebacate, bis(l-octoxy-2,2,6,6-tetramethyl-4-piperidyl)sebacate, tetrakis(2,2,6,6-tetramethyl-4- piperidyl)-l,2,3,4-butanetetracarboxylate, tetrakis(l,2,2,6,6-pentamethyl-4-piperidyl)-l,2,3,4-butanetetracarboxylate, bis(2,2,6,6-tetramethyl-4-piperidyl) di(tridecyl)-l,2,3,4-butanetetracarboxylate, bis(l,2,2,6,6-pentamethyl-4-piperidyl) di(tridecyl)-l,2,3,4-butanetetracarboxylate, bis(l, 2,2,4, 4-pentamethyl-4-piperidyl)-2-butyl-2-(3,5-di-t-4-hydroxybenzyl)malonate, a polycondensate of l-(2-hydroxyethyl)-2,2,6,6-tetramethyl-4-piperidinol and diethyl succinate, a polycondensate of l,6-bis(2,2,6,6-tetramethyl-4-piperidylamino)hexane and 2,4-dichloro-6-morpholino-s-triazine, a polycondensate of 1,6-bis(2,2,6,6-tetramethyl-4-piperidylamino)hexane and 2,4-dichloro-6-t-octylamino-s-triazine, 1.5.8.12-tetrakis[2,4-bis(N-butyl-N-(2,2,6,6-tetramethyl-4-piperidyl)amino)-s-triazin-6-yl]-1.5.8.12-tetraazadodecane, l,5,8,l2-tetrakis[2,4-bis(N-butyl-N-(l,2,2,6,6-pentamethyl-4-piperidyl)amino)-s-triazin-6-yl]-l,5,8-l2 -tetraazadodecane, 1,6,1 l-tris[2,4-bis(N-butyl-N-(2,2,6,6-tetramethyl-4-piperidyl)amino)-s-triazin-6-yl]aminoundecane, and 1,6,1 l-tris[2,4-bis(N- butyl-N-(l,2,2,6,6-pentamethyl-4-piperidyl)amino)-s-triazin-6-yl]aminoundecane.

In some embodiments, the photoresist comprises a quencher, such as a base quencher. The quencher that may be used in the photoresist composition may comprise a weak base that scavenges trace acids, while not having an excessive impact on the performance of the positive photoresist. Illustrative examples of quenchers that can be employed include, but are not limited to: aliphatic amines, aromatic amines, carboxylates, hydroxides, or combinations thereof and the like. Base quenchers may be used in photoresist formulations to improve performance by quenching reactions of photoacids that diffuse into unexposed regions. Base quenchers may comprise aliphatic amines, aromatic amines, carboxylates, hydroxides, or combinations thereof. Examples of base quenchers include but are not limited to, trioctylamine, 1,8-diazabicyclo[5.4.0]undec-7-ene (DBU), 1-piperidineethanol (1PE), tetrabutylammonium hydroxide (TBAH), dimethylamino pyridine, 7-diethylamino-4-methyl coumarin (Coumarin 1), tertiary amines, sterically hindered diamine and guanidine bases such as 1,8-bis(dimethylamino)naphthalene (PROTON SPONGE), berberine, or polymeric amines such as in the PLURONIC or TETRONIC series commercially available from BASF. In some embodiments, the photoresist in the first and the second region comprises the same base quencher. In some embodiments, the photoresist in the first and the second region comprises different base quenchers.

In some embodiments, the photoresist further comprises a photosensitizer. A photosensitizer is a molecule that produces a chemical change in another molecule in a photochemical process. Photosensitizers are commonly used in polymer chemistry in reactions such as photopolymerization, photocrosslinking, and photodegradation. Photosensitizers generally act by absorbing ultraviolet or visible region of electromagnetic radiation and transferring it to adjacent molecules. In some embodiments, photosensitizer shifts the photo sensitivity to a longer wavelength of electromagnetic radiation. The sensitizer, also called a photosensitizer, is capable of activating the photoacid generator (PAG) at, for example, a longer wavelength of light in accordance with an aspect of the present disclosure. In some embodiments, the concentration of the sensitizer is greater than that of the PAG, such as 1.1 times to 5 times greater, for example, 1.1 times to 3 times greater the concentration of PAG. Examples of photosensitizer may include anthracene, N-alkyl carbazole, benzo[a]phenoxazine, and thioxanthone compounds. Exemplary sensitizers suitable for use in the methods disclosed herein include but are not limited to, isopropylthioxanthone (ITX), and 10H-phenoxazine (PhX). In some embodiments, the photoresist in the first and the second region comprises the same photosensitizer. In some embodiments, the photoresist in the first and the second region comprises different photosensitizers. Additional examples of photosensitizers include anthracenes {anthracene, 9,10-dibutoxyanthracene, 9,10-dimethoxyanthracene, 2-ethyl-9,10-dimethoxyanthracene, 2-tert-butyl-9,10-dimethoxyanthracene, 2,3-dimethyl-9,10-dimethoxyanthracene, 9-methoxy-10-methylanthracene, 9,10-diethoxyanthracene, 2-ethyl-9,10-diethoxyanthracene, 2-tert-butyl-9,10-diethoxyanthracene, 2,3-dimethyl-9,10-diethoxyanthracene, 9-ethoxy-10-methylanthracene, 9,10-dipropoxyanthracene, 2-ethyl-9,10-dipropoxyanthracene, 2-tert-butyl-9,10-dipropoxyanthracene, 2,3-dimethyl-9,10-dipropoxyanthracene, 9-isopropoxy-10-methylanthracene, 9,10-dibenzyloxyanthracene, 2-ethyl-9,10-dibenzyloxyanthracene, 2-tert-9,10-dibenzyloxyanthracene, 2,3-dimethyl-9,10-dibenzyloxyanthracene, 9-benzyloxy-10-methylanthracene, 9,10-di-α-methylbenzyloxyanthracene, 2-ethyl-9,10-di-α-methylbenzyloxyanthracene, 2-tert-9,10-di-α-methylbenzyloxyanthracene, 2,3-dimethyl-9,10-di-α-methylbenzyloxyanthracene, 9-(α-methylbenzyloxy)-10-methylanthracene, 9,10-diphenylanthracene, 9-methoxyanthracene, 9-ethoxyanthracene, 9-methylanthracene, 9-bromoanthracene, 9-methylthioanthracene, 9-ethylthioanthracene, and the like}; pyrene; 1,2-benzanthracene; perylene; tetracene; coronene; thioxanthones {thioxanthone, 2-methylthioxanthone, 2-ethylthioxanthone, 2-chlorothioxanthone, 2-isopropylthioxanthone, 2,4-diethylthioxanthone, and the like}; phenothiazine; xanthone; naphthalenes {1-naphthol, 2-naphthol, 1-methoxynaphthalene, 2-methoxynaphthalene, 1,4-dihydroxynaphthalene, 1,5-dihydroxynaphthalene, 1,6-dihydroxynaphthalene, 2,7-dihydroxynaphthalene, 2,7-dimethoxynaphthalene, 1,1'-thiobis(2-naphthol), 1,1'-bis-(2-naphthol), 4-methoxy-1-naphthol, and the like}; ketones {dimethoxyacetophenone, diethoxyacetophenone, 2-hydroxy-2-methyl-1-phenylpropan-1-one, 4'-isopropyl-2-hydroxy-2-methylpropiophenone, 2-hydroxymethyl-2-methylpropiophenone, 2,2-dimethoxy-1,2-diphenylethan-1-one, p-dimethylaminoacetophenone, p-tert-butyldichloroacetophenone, p-tert-butyltrichloroacetophenone, p-azidobenzalacetophenone, 1-hydroxycyclohexyl phenyl ketone, benzoin, benzoin methyl ether, benzoin ethyl ether, benzoin isopropyl ether, benzoin n-dibutyl ether, benzoin isobutyl ether, 1-[4-(2-hydroxyethoxy)phenyl]-2-hydroxy-2-methyl-1-propan-1-one, benzophenone, methyl o-benzoylbenzoate, Michler's ketone, 4,4'-bisdiethylaminobenzophenone, 4,4'-dichlorobenzophenone, 4-benzoyl-4'-methyldiphenylsulfide, and the like}; carbazoles {N-phenylcarbazole, N-ethylcarbazole, poly-N-vinylcarbazole, N-glycidylcarbazole, and the like}; chrysenes {1,4-dimethoxychrysene, 1,4-diethoxychrysene, 1,4-dipropoxychrysene, 1,4-dibenzyloxychrysene, 1,4-di-α-methylbenzyloxychrysene, and the like}; and phenanthrenes {9-hydroxyphenanthrene, 9-methoxyphenanthrene, 9-ethoxyphenanthrene, 9-benzyloxyphenanthrene, 9,10-dimethoxyphenanthrene, 9,10-diethoxyphenanthrene, 9,10-dipropoxyphenanthrene, 9,10-dibenzyloxyphenanthrene, 9,10-di-α-methylbenzyloxyphenanthrene, 9-hydroxy-10-methoxyphenanthrene, 9-hydroxy-10-ethoxyphenanthrene and are described in U.S. Patent Pub. No. 20200384436.

In some embodiments, the photoresist further comprises a matrix. The matrix generally refers to polymeric materials that may provide sufficient adhesion to the substrate when the photoresist formulation is applied to the top surface of the substrate, and may form a substantially uniform film when dissolved in a solvent and deposited on top of a substrate. Examples of a matrix may include, but are not limited to, polyester, polyimide, polyethylene naphthalate (PEN), polyvinyl chloride (PVC), polymethylmethacrylate (PMMA) and polycarbonate, or a combination thereof. The matrix may be chosen based on the wavelength of the radiation used for the generation of acid when using the photoresist formulation, the adhesion properties of the matrix to the top surface of the substrate, the compatibility of the matrix to other components of the formulation, and the ease of removable or degradation (if needed) after use. In some embodiments, the photoresist in the first and the second region comprises the same matrix. In some embodiments, the photoresist in the first and the second region comprises different matrices.

In some embodiments, the photoresist further comprises a surfactant. Surfactants may be used to improve coating uniformity, and may include ionic, non-ionic, monomeric, oligomeric, and polymeric species, or combinations thereof. Examples of possible surfactants include fluorine-containing surfactants such as the FLUORAD series available from 3M Company in St. Paul, Minn., and siloxane-containing surfactants such as the SILWET series available from Union Carbide Corporation in Danbury, Conn. In some embodiments, the photoresist in the first and the second region comprises the same surfactant. In some embodiments, the photoresist in the first and the second region comprises different surfactants.

In some embodiments, the photoresist further comprises a casting solvent. A casting solvent may be used so that the photoresist may be applied evenly on the substrate surface to provide a defect-free coating. Examples of suitable casting solvents may include ethers, glycol ethers, aromatic hydrocarbons, ketones (e.g., methyl ethyl ketone), esters, ethyl lactate, γ-butyrolactone, cyclohexanone, ethoxyethylpropionate (EEP), a combination of EEP and gamma-butyrolactone (GBL), propylene glycol ethyl ether acetate, amyl acetate, propylene glycol methyl ether acetate (PGMEA), and combinations thereof. In some embodiments, the photoresist in the first and the second region comprises the same casting solvent. In some embodiments, the photoresist in the first and the second region comprises different casting solvents. In some embodiments, the solvent may comprise but is not limited to acetone, methyl ethyl ketone, methyl isobutyl ketone, methyl cellosolve, ethyl cellosolve, tetrahydrofuran, 1,4-dioxane, ethylene glycol dimethyl ether, ethylene glycol diethyl ether, propylene glycol dimethyl ether, propylene glycol diethyl ether, diethylene glycol dimethyl ether, diethylene glycol diethyl ether, diethylene glycol methyl ethyl ether, chloroform, methylene chloride, 1,2-dichloroethane, 1,1,1-trichloroethane, 1,1,2-trichloroethane, 1,1,2-trichloroethene, hexane, heptane, octane, cyclohexane, benzene, toluene, xylene, methanol, ethanol, isopropanol, propanol, butanol, t-butanol, 2-ethoxypropanol, 2-methoxypropanol, 3-methoxybutanol, cyclohexanone, cyclopentanone, propylene glycol methyl ether acetate, propylene glycol ethyl ether acetate, 3-methoxybutyl acetate, ethyl 3-ethoxypropionate, ethyl cellosolve acetate, methyl cellosolve acetate, butyl acetate, propylene glycol monomethyl ether, or dipropylene glycol monomethyl ether, or any combination thereof. In some embodiments, the solvent may comprise any one or more of those selected from the group consisting of ketones such as γ-butyrolactone, 1,3-dimethyl-imidazolidinone, methyl ethyl ketone, cyclohexanone, cyclopentanone and 4-hydroxy-4-methyl-2-pentanone; aromatic hydrocarbons such as toluene, xylene and tetramethylbenzene; glycol ethers (cellosolve) such as ethylene glycol monoethyl ether, ethylene glycol monomethyl ether, ethylene glycol monobutyl ether, diethylene glycol monoethyl ether, diethylene glycol monomethyl ether, diethylene glycol monobutyl ether, propylene glycol monomethyl ether, propylene glycol monoethyl ether, dipropylene glycol diethyl ether and triethylene glycol monoethyl ether; ethyl acetate, butyl acetate, ethylene glycol monoethyl ether acetate, ethylene glycol monobutyl ether acetate, diethylene glycol monoethyl ether acetate, dipropylene glycol monomethyl ether acetate, propylene glycol monomethyl ether acetate (PGMEA), ethanol, propanol, ethylene glycol, propylene glycol, carbitol, dimethylacetamide (DMAc), N,N-diethylacetamide, dimethylformamide (DMF), diethylformamide (DEF), N,N-dimethylacetamide (DMAc), N-methylpyrrolidone (NMP), N-ethylpyrrolidone (NEP), 1,3-dimethyl-2-imidazolidinone, N,N-dimethylmethoxyacetamide, dimethyl sulfoxide, pyridine, dimethyl sulfone, hexamethylphosphoramide, tetramethylurea, N-methylcaprolactam, tetrahydrofuran, m-dioxane, p-dioxane, 1,2-dimethoxyethane, bis(2-methoxyethyl)ether, 1,2-bis(2-methoxyethoxy)ethane, bis[2-(2-methoxyethoxy)]ether, and mixtures thereof. Examples of solvents for use in photoresists are described in U.S. Patent Pub. No. 20200384436 and U.S. Patent Pub. No. 20210017127.

In some embodiments of aspects provided herein, the photoresist composition comprises: the photoacid generator: about 1-10% (e.g., about 2-5%) by weight; the photosensitizer: about about 1-10% (e.g., 2-5%) by weight; an acid scavenger: about 0.1-0.5% by weight; a matrix: about 2.5-4.5% by weight; and a solvent. In some embodiments of aspects provided herein, the photoresist composition comprises: the photoacid generator: about 2.5-4.5% by weight; the photosensitizer: about 2.5-4.5% by weight; the acid scavenger: about 0.15-0.35% by weight; the matrix: about 3.0-4.0% by weight; and the solvent. In some embodiments of aspects provided herein, weight percentage of the photosensitizer is substantially the same as weight percentage of the photoacid generator. In some embodiments of aspects provided herein, the weight percentage of the photosensitizer is the same as the weight percentage of the photoacid generator. Suitable photoresist compositions are described, for example, in U.S. Patent Pub. No. 20200384436.

Methods of applying photoresist to the substrate include, but are not limited to, dipping, spreading, spraying, or any combination thereof. In some embodiments, the photoresist is applied via spin coating, thereby forming a photoresist layer on the substrate.

In some embodiments, the photoresist is in direct contact with the oligonucleotides on the substrate. In some embodiments, the oligonucleotide molecules on the substrate are embedded in the photoresist. In some embodiments, the photoresist is not in direct contact with the oligonucleotides. In some embodiments, oligonucleotide molecules on the substrate are embedded in an underlayer that is underneath the photoresist. For example, oligonucleotide molecules on the substrate may be embedded in a soluble polymer underlayer (e.g., a soluble polyimide underlayer (XU-218)), and the photoresist forms a photoresist layer on top of the underlayer.

In some embodiments, the photoresist may be removed and re-applied. For example, the photoresist may be stripped from the substrate and/or the oligonucleotides ligated to the substrate. Removal of photoresist can be accomplished with various degrees of effectiveness. In some embodiments, the photoresist is completely removed from the substrate and/or the oligonucleotides ligated to the substrate before re-application. Methods of removing photoresist may include, but are not limited to, using organic solvent mixtures, using liquid chemicals, exposure to a plasma environment, or other dry techniques such as UV/O₃ exposure. In some embodiments, the photoresist is stripped using organic solvent. In some embodiments, the photoresist may be removed after each cycle of *in situ* array generation and re-applied prior to the next cycle of *in situ* array generation. In some embodiments, the photoresist removed, and the photoresist re-applied prior to the next cycle is the same photoresist. In some embodiments, the photoresist removed, and the photoresist re-applied prior to the next cycle are different photoresists.

In some embodiments, one or more photomasks also referred to herein as "masks" may be used to selectively remove photoresist on the substrate. The mask is designed in such a way that light exposed sites can be selected, and thus specify the coordinates on the array where oligonucleotide molecules can be blocked or ablated (e.g., digested). In some embodiments, after blocking or digesting the oligonucleotides in the first region (e.g., the boundary region), the method comprises ligating one or more oligonucleotides to the oligonucleotide molecules in the second region (e.g., one or more features or spots) to generate extended oligonucleotide molecules in the second region. This process can be used to synthesize hundreds of thousands or millions of different oligonucleotides. In some embodiments, the ligation steps also comprise irradiating the substrate through a patterned mask that is different than the patterned mask used to specify the coordinates on the array where oligonucleotide molecules are blocked or digested. In some embodiments, the patterned mask used for ligation steps exposes the features or spots for a given ligation cycle. The mask may be an opaque plate or film with transparent areas that allow light to shine through in a pre-defined pattern. After the irradiation step, the mask may be removed, translated to a different region on the substrate, or rotated. Using a series of photomasks, photoresist in desired regions of the substrate may be iteratively irradiated and subsequently removed. In some embodiments, the irradiation to block or ablate oligonucleotide molecules in the boundary region is performed in a single step, and irradiations to expose the features or spots for a given ligation cycle is performed in multiple cycles.

The material of the photomask used herein may comprise silica with chrome in the opaque part. For example, the photomask may be transparent fused silica blanks covered with a pattern defined with a chrome metal absorbing film. The photomask may be used at various irradiation wavelengths, which include but are not limited to, 365 nm, 248 nm, and 193 nm. In some embodiments, the irradiation step herein can be performed for a duration of between about 1 minute and about 10 minutes, for example, for about 2 minutes, about 4 minutes, about 6 minutes, or about 8 minutes. In some embodiments, the irradiation can be performed at a total light dose of between about one and about ten mW/mm², for example, at about 2 mW/mm², about 4 mW/mm², about 6 mW/mm², or about 8 mW/mm². In some embodiments, the irradiation can be performed at a total light dose of between about one and about ten mW/mm² and for a duration of between about 1 minute and about 10 minutes.

### III. HYBRIDIZATION/LIGATION

In some embodiments, the methods comprised herein comprise attaching one or more nucleotide barcode parts to oligonucleotide molecules immobilized in features on the substrate, wherein oligonucleotide molecules in the boundary regions are blocked or ablated (e.g., digested). The nucleotide barcode parts described herein may be linked via phosphodiester bonds. The nucleotide barcode parts may also be linked via non-natural oligonucleotide linkages such as methylphosphonate or phosphorothioate bonds, via non-natural biocompatible linkages such as click-chemistry, via enzymatic biosynthesis of nucleic acid polymers such as by polymerase or transcriptase, or a combination thereof. Ligation may be achieved using methods that include, but are not limited to, primer extension, hybridization ligation, enzymatic ligation, and chemical ligation. In some embodiments, the oligonucleotide comprising the barcode sequence is hybridized to a splint which is in turn hybridized to an oligonucleotide molecule in an unmasked region. The oligonucleotide comprising the barcode sequence may be further ligated to the oligonucleotide in the unmasked region to generate a barcoded oligonucleotide molecule.

In some cases, a primer extension or other amplification reaction may be used to synthesize an oligonucleotide on a substrate via a primer attached to the substrate. In such cases, a primer attached to the substrate may hybridize to a primer binding site of an oligonucleotide that also contains a template nucleotide sequence. The primer can then be extended by a primer extension reaction or other amplification reaction, and an oligonucleotide complementary to the template oligonucleotide can thereby be attached to the substrate.

In some embodiments, chemical ligation can be used to ligate two or more oligonucleotides. In some embodiments, chemical ligation involves the use of condensing reagents. In some embodiments, condensing reagents are utilized to activate a phosphate group. In some embodiments, condensing reagents may be one or more of 1-ethyl-3-(3-dimethylaminopropyl) carbodiimide (EDCI), cyanogen bromide, imidazole derivatives, and 1-hydroxybenzotriazole (HOAt). In some embodiments, functional group pairs selected from one or more of a nucleophilic group and an electrophilic group, or an alkyne and an azide group are used for chemical ligation. In some embodiments, chemical ligation of two or more oligonucleotides requires a template strand that is complementary to the oligonucleotides to be ligated (e.g., a splint).

A splint is an oligonucleotide that, when hybridized to other polynucleotides, acts as a "splint" to position the polynucleotides next to one another so that they can be ligated together. In some embodiments, the splint is DNA or RNA. The splint can include a nucleotide sequence that is partially complimentary to nucleotide sequences from two or more different oligonucleotides. In some embodiments, the splint assists in ligating a "donor" oligonucleotide and an "acceptor" oligonucleotide. In general, an RNA ligase, a DNA ligase, or another other variety of ligase is used to ligate two nucleotide sequences together.

Splints have been described, for example, in US20150005200A1. A splint may be used for ligating two oligonucleotides. The sequence of a splint may be configured to be in part complementary to at least a portion of the first oligonucleotides that are attached to the substrate and in part complementary to at least a portion of the second oligonucleotides. In one case, the splint can hybridize to the second oligonucleotide via its complementary sequence; once hybridized, the second oligonucleotide or oligonucleotide segment of the splint can then be attached to the first oligonucleotide attached to the substrate via any suitable attachment mechanism, such as, for example, a ligation reaction. The splint complementary to both the first and second oligonucleotides can then be then denatured (or removed) with further processing. The method of attaching the second oligonucleotides to the first oligonucleotides can then be optionally repeated to ligate a third, and/or a fourth, and/or more parts of the barcode onto the array with the aid of splint(s). In some embodiments, the splint is between 6 and 50 nucleotides in length, e.g., between 6 and 45, 6 and 40, 6 and 35, 6 and 30, 6 and 25, or 6 and 20 onucleotides in length. In some embodiments, the splint is between 15 and 50, 15 and 45, 15 and 40, 15 and 35, 15 and 30, 15 and 30, or 15 and 25 nucleotides in length.

In some embodiments, the method for providing an array described herein comprises irradiating the substrate to render an oligonucleotide molecule immobilized in a first one or more of the spot regions on the substrate available for oligonucleotide attachment, whereas an oligonucleotide molecule immobilized in one or more other spot regions on the substrate are not available for oligonucleotide attachment; and attaching a first oligonucleotide of at least four nucleotides in length to the oligonucleotide molecule immobilized in the first one or more of the spot regions to provide an extended oligonucleotide molecule in the first one or more of the spot regions. In some embodiments, the first oligonucleotide of at least four nucleotides in length is attached to the oligonucleotide molecule immobilized in the first one or more of the spot regions by a ligation using a first splint. In some embodiments, the first splint hybridizes to the first oligonucleotide and the oligonucleotide molecules in the first region. In some embodiments, the first oligonucleotide is not ligated to oligonucleotide molecules in the second region. In some embodiments, the hybridization region between the first splint and the oligonucleotide molecules is at least 3, 4, 5, 6, 7, 8, 9, 10 bp or more than 10 bp. In some embodiments, the hybridization region between the first splint and the first oligonucleotide is at least 3, 4, 5, 6, 7, 8, 9, 10 bp or more than 10 bp.

In some embodiments, the oligonucleotide is ligated using the splint as template without gap filling prior to the ligation. In some embodiments, the oligonucleotide is ligated using the splint as template with gap filling prior to the ligation. In some embodiments, hybridization to the first splint brings the terminal nucleotides of the first oligonucleotide and the oligonucleotide molecules immediately next to each other, and the ligation does not require gap-filling. In some embodiments, hybridization to the first splint brings the terminal nucleotides of the first oligonucleotide and the oligonucleotide molecules next to each other and separated by one or more nucleotides, and the ligation is preceded by gap-filling. In some embodiments, the splint is removed after the ligation.

In some embodiments, the oligonucleotide molecule in a given spot is ligated to one or more oligonucleotide molecules to generate an oligonucleotide probe for capturing analytes or proxies thereof. In some embodiments, an array containing a plurality of oligonucleotides (*e.g., in situ* synthesized oligonucleotides) can be modified to generate a variety of oligonucleotide probes. The oligonucleotides can include various domains such as, spatial barcodes, UMIs, functional domains (*e.g*., sequencing handle), cleavage domains, and/or ligation handles.

A "spatial barcode" may comprise a contiguous nucleic acid segment or two or more non-contiguous nucleic acid segments that function as a label or identifier that conveys or is capable of conveying spatial information. In some embodiments, a capture probe includes a spatial barcode that possesses a spatial aspect, where the barcode is associated with a particular location within an array or a particular location on a substrate. A spatial barcode can be part of a capture probe on an array generated herein. A spatial barcode can also be a tag attached to an analyte (e.g., a nucleic acid molecule) or a combination of a tag in addition to an endogenous characteristic of the analyte (e.g., size of the analyte or end sequence(s)). A spatial barcode can be unique. In some embodiments where the spatial barcode is unique, the spatial barcode functions both as a spatial barcode and as a unique molecular identifier (UMI), associated with one particular capture probe. Spatial barcodes can have a variety of different formats. For example, spatial barcodes can include polynucleotide spatial barcodes; random nucleic acid and/or amino acid sequences; and synthetic nucleic acid and/or amino acid sequences. In some embodiments, a spatial barcode is attached to an analyte in a reversible or irreversible manner. In some embodiments, a spatial barcode is added to, for example, a fragment of a DNA or RNA sample before sequencing of the sample. In some embodiments, a spatial barcode allows for identification and/or quantification of individual sequencing-reads. In some embodiments, a spatial barcode is a used as a fluorescent barcode for which fluorescently labeled oligonucleotide probes hybridize to the spatial barcode.

In some embodiments, a spatial array is generated after ligating capture domains *(e.g.,* poly(T) or gene specific capture domains) to the oligonucleotide molecule *(e.g.,* generating capture oligonucleotides). The spatial array can be used with any of the spatial analysis methods described herein. For example, a biological sample (e.g., a tissue section) can be provided to the generated spatial array. In some embodiments, the biological sample is permeabilized. In some embodiments, the biological sample is permeabilized under conditions sufficient to allow one or more analytes or proxies thereof present in the biological sample to interact with the capture probes of the spatial array. After capture of analytes or proxies thereof from the biological sample, the analytes can be analyzed (*e.g*., reverse transcribed, amplified, and/or sequenced) by any of the variety of methods described herein.

In some embodiments, prior to the oligonucleotide blocking or ablation step, the substrate comprises a uniform lawn of oligonucleotide molecules. In some embodiments, the oligonucleotide molecules are about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 30, about 35, about 40, about 45, about 50, about 55, about 60, about 65, or about 70 nucleotides in length. In some embodiments, the oligonucleotide molecules on the substrate are between about 4 and about 50 nucleotides in length. In some embodiments, the oligonucleotide molecules comprise a primer sequence. In some embodiments, the primer sequence is a common sequence among the oligonucleotide molecules in the lawn. In some embodiments, the primer sequence is a sequencing handle that comprises a primer binding site for subsequent processing. The primer sequence can generally be selected for compatibility with any of a variety of different sequencing systems, e.g., 454 Sequencing, Ion Torrent Proton or PGM, Illumina X10, PacBio, Nanopore, etc., and the requirements thereof. In some embodiments, functional sequences can be selected for compatibility with non-commercialized sequencing systems. Examples of such sequencing systems and techniques, for which suitable functional sequences can be used, include (but are not limited to) Roche 454 sequencing, Ion Torrent Proton or PGM sequencing, Illumina X10 sequencing, PacBio SMRT sequencing, and Oxford Nanopore sequencing. Further, in some embodiments, functional sequences can be selected for compatibility with other sequencing systems, including non-commercialized sequencing systems. In some embodiments the common sequence is about 10, about 11, about 12, about 13, about 14, about 15, about 16, about 17, about 18, about 19, about 20, about 21, about 21, about 22, about 23, about 24, about 25, about 26, about 27, about 28, about 29, about 30, about 31, about 32, about 33, about 34, or about 35 nucleotides in length. In some embodiments, the common sequence is between about 10 and about 35 nucleotides in length.

In some embodiments, after a step of blocking or ablating oligonucleotide molecules in the boundary region between feartures, or before a step of ablating oligonucleotide molecules in the boundary region between features, an oligonucleotide comprising a part of a barcode (*e.g.,* BC-A) is attached to the oligonucleotide molecule comprising the primer (e.g., R1 primer). In some embodiments, the barcode part can be common to all of the oligonucleotide molecules in a given feature. In some embodiments, the barcode part can be common to all of the oligonucleotide molecules in multiple substrate regions (e.g., features) in the same cycle. In some embodiments, the barcode part can be different for oligonucleotide molecules in different substrate regions (e.g., features) in different cycles. In some embodiments, a splint with a sequence complementary to a portion of the primer of the immobilized oligonucleotide and an additional sequence complementary to a portion of the oligonucleotide comprising the part of the barcode (e.g., BC-A) facilitates the ligation of the immobilized oligonucleotide and the oligonucleotide comprising BC-A. In some embodiments, the splint for attaching the part of the barcode (e.g., BC-A) of various sequences to different substrate regions (e.g., features) is common among the cycles of the same round. In some embodiments, the splint for attaching the part of the barcode (e.g., BC-A) of various sequences to different substrate regions (e.g., features) can be different among the cycles of the same round. In some embodiments, the splint for attaching the part of the barcode may comprise a sequence complementary to the part (e.g., BC-A) or a portion thereof.

In some embodiments, another cycle, such as a second cycle, of photohybridization/ligation involves the addition of another oligonucleotide comprising another part of a barcode (*e.g.,* BC-B) to the immobilized oligonucleotide molecule comprising the primer and BC-A. In some embodiments, a splint with a sequence complementary to a portion of the immobilized oligonucleotide comprising BC-A and an additional sequence complementary to a portion of the oligonucleotide comprising BC-B facilitates the ligation of the oligonucleotide comprising BC-B and the immobilized oligonucleotide comprising BC-A. In some embodiments, the splint for attaching part BC-B of various sequences to different substrate regions (e.g., features) is common among the cycles of the same round. In some embodiments, the splint for attaching BC-B to different substrate regions (e.g., features) can be different among the cycles of the same round. In some embodiments, the splint for attaching BC-B may comprise a sequence complementary to BC-B or a portion thereof and/or a sequence complementary to BC-A or a portion thereof. In some embodiments, the photo-hybridization ligation is performed using exemplary methods and reagents described in US 2022/0228201 A1, US 2022/0228210 A1, and US 2022/0314187 A1.

In some embodiments, the method comprises a third cycle of photohybridization/ligation, which involves the addition of another oligonucleotide comprising another part of a barcode (*e.g*., BC-C), added to the immobilized oligonucleotide molecule comprising the primer, BC-A, and BC-B. In some embodiments, a splint with a sequence complementary to a portion of the immobilized oligonucleotide molecule comprising BC-B and an additional sequence complementary to a portion of the oligonucleotide comprising BC-C facilitates the ligation of the immobilized oligonucleotide molecule comprising BC-B and the oligonucleotide comprising BC-C. In some embodiments, the splint for attaching part BC-C of various sequences to different substrate regions (e.g., features) is common among the cycles of the same round. In some embodiments, the splint for attaching BC-C to different substrate regions (e.g., features) can be different among the cycles of the same round. In some embodiments, the splint for attaching BC-C may comprise a sequence complementary to BC-C or a portion thereof and/or a sequence complementary to BC-B or a portion thereof. In some embodiments, the photo-hybridization ligation is performed using exemplary methods and reagents described in US 2022/0228201 A1, US 2022/0228210 A1, and US 2022/0314187 A1.

A fourth cycle of photo-hybridization/ligation may be performed, which involves the addition of another oligonucleotide comprising another part of a barcode *(e.g.,* BC-D), added to the immobilized oligonucleotide molecule comprising the primer, BC-A, BC-B, and BC-C. In some embodiments, a splint with a sequence complementary to a portion of the immobilized oligonucleotide molecule comprising BC-C and an additional sequence complementary to a portion of the oligonucleotide comprising BC-D facilitates the ligation. In some embodiments, the splint for attaching part BC-D of various sequences to different substrate regions (e.g., features) is common among the cycles of the same round. In some embodiments, the splint for attaching BC-D to different substrate regions (e.g., features) can be different among the cycles of the same round. In some embodiments, the splint for attaching BC-D may comprise a sequence complementary to BC-D or a portion thereof and/or a sequence complementary to BC-C or a portion thereof. In some embodiments, an oligonucleotide comprising BC-D further comprises a UMI and a capture domain. In some embodiments, the photo-hybridization ligation is performed using exemplary methods and reagents described in US 2022/0228201 A1, US 2022/0228210 A1, and US 2022/0314187 A1.

In some embodiments, the splint comprises a sequence that is complementary to an oligonucleotide (*e.g*., an immobilized oligonucleotide), or a portion thereof, and a sequence that is complementary to an oligonucleotide containing a barcode, or a portion thereof. In some embodiments, the splint comprises a sequence that is perfectly complementary (*e.g*., is 100% complementary) to an oligonucleotide (*e.g*., an immobilized oligonucleotide), or a portion thereof, and/or a sequence that is perfectly complementary to an oligonucleotide containing a barcode, or a portion thereof. In some embodiments, the splint comprises a sequence that is not perfectly complementary *(e.g.,* is not 100% complementary) to an oligonucleotide *(e.g.,* an immobilized oligonucleotide), or a portion thereof, and/or a sequence that is not perfectly complementary to an oligonucleotide containing a barcode, or a portion thereof. In some embodiments, the splint comprises a sequence that is at least 70%, 75%, 80%, 85%, 90%, 95%, 96%, 97%, 98%, or 99% complementary to an oligonucleotide (*e.g*., an immobilized oligonucleotide), or a portion thereof, and/or a sequence that is complementary to an oligonucleotide containing a barcode, or a portion thereof. In some embodiments, the splint comprises a sequence that is perfectly complementary (e.g., is 100% complementary) to an oligonucleotide (*e.g*., an immobilized oligonucleotide), or a portion thereof, but is not perfectly complementary to a sequence that is complementary to an oligonucleotide containing a barcode, or a portion thereof. In some embodiments, the splint comprises a sequence that is not perfectly complementary *(e.g.,* is not 100% complementary) to an oligonucleotide *(e.g.,* an immobilized oligonucleotide), or a portion thereof, but is perfectly complementary to a sequence that is complementary to an oligonucleotide containing a barcode, or a portion thereof. So long as the splint is capable of hybridizing to an oligonucleotide (*e.g*., an immobilized oligonucleotide), or a portion thereof, and to a sequence that is complementary to an oligonucleotide containing a barcode, or a portion thereof, the splint need not have a sequence that is perfectly complementary to either the oligonucleotide (*e.g*., the immobilized oligonucleotide) or to the oligonucleotide containing a barcode.

In some embodiments, oligonucleotides that are exposed and do not receive a ligated oligonucleotide could receive the incorrect barcode during the next cycle or round. In order to prevent generating the wrong barcode at the wrong feature, unligated oligonucleotides may be rendered unavailable for hybridization and/or ligation, *e.g.,* the unligated oligonucleotides can be capped and/or removed. In some embodiments, the oligonucleotides are modified at the 3' termini. Non-limiting examples of 3' modifications include dideoxy C-3' (3'-ddC), 3' inverted dT, 3' C3 spacer, 3'Amino, and 3' phosphorylation.

In some embodiments according to any of the methods described herein, the method further comprises blocking the 3' or 5' termini of barcoded oligonucleotide molecules. In some embodiments, the method further comprises blocking the unligated oligonucleotide molecules in the first region from ligation. In some embodiments, the blocking comprises adding a 3' dideoxy or a non-ligating 3' phosphoramidite to the barcoded oligonucleotide molecules. In some embodiments, the blocking comprises adding a 3' dideoxy or a non-ligating 3' phosphoramidite to the unligated oligonucleotide molecules. In some embodiments, the addition is catalyzed by a terminal transferase. In some embodiments, the terminal transferase is a terminal deoxynucleotidyl transferase (TdT). The blocking may be removed after the blocking reaction is completed. In some embodiments, the blocking is removed using an internal digestion of the barcoded oligonucleotide molecules after ligation is completed.

### IV. PHOTOLITHOGRAPHY AND LIGHT-CONTROLLED LIGATION

Provided herein in some embodiments are methods and uses of photohybridization-ligation combinatorial barcode generation using any suitable light-controlled surface patterning (e.g., photoresist, polymers, caged oligonucleotides, etc.) and photolithography. For example, a method disclosed herein may comprise photocontrollable ligation, wherein local irradiation causes degradation of photoresist and oligonucleotides to be exposed for ligation. In some aspects, a method disclosed herein provides one or more advantages as compared to available arraying methods. For example, a large diversity of barcodes can be created via sequential rounds of UV exposure, hybridization, ligation, removal and reapplication using light-controlled surface patterning; no protection/deprotection step is required for ligating oligonucleotides to the substrate; the feature size can be highly controlled using photomasks, and the generated array at any discrete location is known and across all arrays with no decoding needed.

The methods of the present disclosure comprise irradiating a substrate to render oligonucleotide molecules in one or more regions on the substrate available for oligonucleotide attachment, whereas oligonucleotide molecules in one or more other regions on the substrate are not available for oligonucleotide attachment. In some embodiments, the irradiation is selective, for example, where one or more photomasks can be used such that only one or more specific regions of the array are exposed to stimuli (*e.g*., exposure to light such as UV, and/or exposure to heat induced by laser). In some embodiments, the method comprises irradiating oligonucleotide molecules in one or more regions with a first light while oligonucleotide molecules in one or more other regions are not irradiated with the first light. For instance, the substrate is exposed to the first light when the oligonucleotide molecules in the one or more other regions are photomasked while the oligonucleotide molecules in the one or more regions are not photomasked. Alternatively, a focused light such as laser may be used to irradiate the oligonucleotide molecules in the one or more regions but not the oligonucleotide molecules in the one or more other regions, even when the oligonucleotide molecules in the one or more other regions are not masked from the light. For example, the distance (pitch) between features may be selected to prevent the laser from stimulating oligonucleotides of an adjacent feature.

In some embodiments, during and after the irradiation, the oligonucleotide molecules in the one or more other regions are protected from hybridization (e.g., hybridization to a splint and/or an oligonucleotide molecule). In some embodiments, during and after the irradiation, the oligonucleotide molecules in the one or more other regions are protected from ligation (e.g., hybridization to a splint and/or an oligonucleotide molecule). In some embodiments, during and after the irradiation, the oligonucleotide molecules in the one or more other regions are protected from hybridization and ligation. Various strategies for the protection of the oligonucleotides from hybridization and/or ligation are contemplated herein.

As described herein, the immobilized nucleic acid generated using a method provided herein can provide a higher resolution and/or higher barcode accuracy compared to certain methods of light mediated base-by-base *in situ* synthesis.

In some aspects, the methods provided herein comprise attaching oligonucleotides (e.g. a barcode) to a substrate. Oligonucleotides may be attached to the substrate according to the methods set forth in U.S. Patent Nos. 6,737,236, 7,259,258, 7,375,234, 7,309,593, 7,427,678, 5,610,287, 5,807,522, 5,837,860, and 5,472,881; U.S. Patent Application Publication Nos. 2008/0280773, 2011/0143967, and 2011/0059865; Shalon et al. (1996) Genome Research, 639-645; Rogers et al. (1999) Analytical Biochemistry 266, 23-30; Stimpson et al. (1995) Proc. Natl. Acad. Sci. USA 92, 6379-6383; Beattie et al. (1995) Clin. Chem. 45, 700-706; Lamture et al. (1994) Nucleic Acids Research 22, 2121-2125; Beier et al. (1999) Nucleic Acids Research 27, 1970-1977; Joos et al. (1997) Analytical Biochemistry 247, 96-101; Nikiforov et al. (1995) Analytical Biochemistry 227, 201-209; Timofeev et al. (1996) Nucleic Acids Research 24, 3142-3148; Chrisey et al. (1996) Nucleic Acids Research 24, 3031-3039; Guo et al. (1994) Nucleic Acids Research 22, 5456-5465; Running and Urdea (1990) BioTechniques 8, 276-279; Fahy et al. (1993) Nucleic Acids Research 21, 1819-1826; Zhang *et al.* (1991) 19, 3929-3933; and Rogers et al. (1997) Gene Therapy 4, 1387-1392.

In some embodiments, oligonucleotides may be immobilized by spotting (e.g., DNA printing) on a substrate with reactive surface chemistry, such as a polymer (e.g., a hydrophilic polymer) containing epoxy reactive groups. In some embodiments, the polymer comprises a passivating polymer. In some embodiments, the polymer comprises a photoreactive group for attachment to the substrate (such as a glass slide). In some embodiments, the oligonucleotides may be immobilized in a DNA printing buffer, optionally wherein the printing buffer comprises a surfactant such as sarcosyl (e.g., a buffer containing sodium phosphate and about 0.06% sarcosyl). In some embodiments, after immobilization of the oligonucleotides, one or more wash and/or blocking steps are performed. Blocking steps can comprise contacting the substrate with a solution that deactivates or blocks unreacted functional groups on the substrate surface. In one example, the blocking buffer can comprise ethanolamine (e.g., to deactivate epoxy silane or other epoxy reactive functional groups).

Arrays can be prepared by a variety of methods. In some embodiments, arrays are prepared through the synthesis (e.g., *in situ* synthesis) of oligonucleotides on the array, or by jet printing or lithography. For example, light-directed synthesis of high-density DNA oligonucleotides can be achieved by photolithography or solid-phase DNA synthesis. To implement photolithographic synthesis, synthetic linkers modified with photochemical protecting groups can be attached to a substrate and the photochemical protecting groups can be modified using a photolithographic mask (applied to specific areas of the substrate) and light, thereby producing an array having localized photo-deprotection. Many of these methods are known in the art, and are described e.g., in Miller et al., "Basic concepts of microarrays and potential applications in clinical microbiology." Clinical microbiology reviews 22.4 (2009): 611-633; US201314111482A; US9593365B2; US2019203275; and WO2018091676.

In some embodiments, a substrate comprising an array of molecules is provided, e.g., in the form of a lawn of polymers (e.g., oligonucleotides), or polymers on the substrate in a pre-determined pattern. In some embodiments, prior to the oligonucleotide blocking or ablation step, a substrate comprising an array of molecules is provided in the form of a lawn of polymers (e.g. oligonucleotides). In some aspects, the oligonucleotide blocking or ablation step provides precisely defined features (e.g., spots of immobilized oligonucleotides that are not blocked or ablated) in a pre-determined pattern, wherein oligonucleotide molecules outside of the features (e.g., in the boundary region) have been blocked or ablated.

Examples of polymers on an array may include, but are not limited to, nucleic acids, peptides, phospholipids, polysaccharides, heteromacromolecules in which a known drug is covalently bound to any of the above, polyurethanes, polyesters, polycarbonates, polyureas, polyamides, polyethyleneimines, polyarylene sulfides, polysiloxanes, polyimides, and polyacetates. The molecules occupying different features of an array typically differ from one another, although some redundancy in which the same polymer occupies multiple features can be useful as a control. For example, in a nucleic acid array, the nucleic acid molecules within the same feature are typically the same, whereas nucleic acid molecules occupying different features are mostly different from one another.

In some examples, the molecules on the array may be nucleic acids. The nucleic acid molecule can be single-stranded or double-stranded. Nucleic acid molecules on an array may be DNA or RNA. The DNA may be single-stranded or double-stranded. The DNA may include, but are not limited to, mitochondrial DNA, cell-free DNA, complementary DNA (cDNA), genomic DNA, plasmid DNA, cosmid DNA, bacterial artificial chromosome (BAC), or yeast artificial chromosome (YAC). The RNA may include, but are not limited to, mRNAs, tRNAs, snRNAs, rRNAs, retroviruses, small non-coding RNAs, microRNAs, polysomal RNAs, pre-mRNAs, intronic RNA, viral RNA, cell free RNA and fragments thereof. The non-coding RNA, or ncRNA can include snoRNAs, microRNAs, siRNAs, piRNAs and long non-coding RNAs (lncRNAs).

In some embodiments, the molecules on an array comprise oligonucleotide barcodes. A barcode sequence can be of varied length. In some embodiments, the barcode sequence is about 3, about 4, about 5, about 6, about 7, about 8, about 9, about 10, about 11, about 12, about 13, about 14, about 15, about 16, about 17, about 18, about 19, about 20, about 21, about 22, about 23, about 24, about 25, about 30, about 35, about 40, about 45, about 50, about 55, about 60, about 65, or about 70 nucleotides in length. In some embodiments, the barcode sequence is between about 4 and about 25 nucleotides in length. In some embodiments, the barcode sequences is between about 10 and about 50 nucleotides in length. The nucleotides can be completely contiguous, i.e., in a single stretch of adjacent nucleotides, or they can be separated into two or more separate subsequences that are separated by 1 or more nucleotides. In some embodiments, the barcode sequence can be about 4, about 5, about 6, about 7, about 8, about 9, about 10, about 11, about 12, about 13, about 14, about 15, about 16, about 17, about 18, about 19, about 20, about 21, about 22, about 23, about 24, about 25 nucleotides or longer. In some embodiments, the barcode sequence can be at least about 4, about 5, about 6, about 7, about 8, about 9, about 10, about 11, about 12, about 13, about 14, about 15, about 16, about 17, about 18, about 19, about 20, about 21, about 22, about 23, about 24, about 25 nucleotides or longer. In some embodiments, the barcode sequence can be at most about 4, about 5, about 6, about 7, about 8, about 9, about 10, about 11, about 12, about 13, about 14, about 15, about 16, about 17, about 18, about 19, about 20, about 21, about 22, about 23, about 24, about 25 nucleotides or shorter.

The oligonucleotide can include one or more (e.g., two or more, three or more, four or more, five or more) Unique Molecular Identifiers (UMIs). A unique molecular identifier is a contiguous nucleic acid segment or two or more non-contiguous nucleic acid segments that function as a label or identifier for a particular analyte, or for a capture probe that binds a particular analyte (e.g., via the capture domain).

A UMI can be unique. A UMI can include one or more specific polynucleotides sequences, one or more random nucleic acid and/or amino acid sequences, and/or one or more synthetic nucleic acid and/or amino acid sequences.

In some embodiments, the UMI is a nucleic acid sequence that does not substantially hybridize to analyte nucleic acid molecules in a biological sample. In some embodiments, the UMI has less than 90% sequence identity (e.g., less than 80%, 70%, 60%, 50%, or less than 40% sequence identity) to the nucleic acid sequences across a substantial part (e.g., 80% or more) of the nucleic acid molecules in the biological sample.

The UMI can include from about 6 to about 20 or more nucleotides within the sequence of capture probes, e.g., barcoded oligonucleotides in an array generated using a method disclosed herein. In some embodiments, the length of a UMI sequence can be about 6, 7, 8, 9, 10, 11, 12, 13, 14, 15, 16, 17, 18, 19, 20 nucleotides or longer. In some embodiments, the length of a UMI sequence can be at least about 6, 7, 8, 9, 10, 11, 12, 13, 14, 15, 16, 17, 18, 19, 20 nucleotides or longer. In some embodiments, the length of a UMI sequence is at most about 6, 7, 8, 9, 10, 11, 12, 13, 14, 15, 16, 17, 18, 19, 20 nucleotides or shorter. These nucleotides can be contiguous, i.e., in a single stretch of adjacent nucleotides, or they can be separated into two or more separate subsequences that are separated by 1 or more nucleotides. Separated UMI subsequences can be from about 4 to about 16 nucleotides in length. In some embodiments, the UMI subsequence can be about 4, 5, 6, 7, 8, 9, 10, 11, 12, 13, 14, 15, 16 nucleotides or longer. In some embodiments, the UMI subsequence can be at least about 4, 5, 6, 7, 8, 9, 10, 11, 12, 13, 14, 15, 16 nucleotides or longer. In some embodiments, the UMI subsequence can be at most about 4, 5, 6, 7, 8, 9, 10, 11, 12, 13, 14, 15, 16 nucleotides or shorter.

In some embodiments, a UMI is attached to other parts of the oligonucleotide in a reversible or irreversible manner. In some embodiments, a UMI is added to, for example, a fragment of a DNA or RNA sample before sequencing of the analyte. In some embodiments, a UMI allows for identification and/or quantification of individual sequencing-reads. In some embodiments, a UMI is used as a fluorescent barcode for which fluorescently labeled oligonucleotide probes hybridize to the UMI.

In some embodiments, a method provided herein further comprises a step of providing the substrate. A wide variety of different substrates can be used for the foregoing purposes. In general, a substrate can be any suitable support material. The substrate may comprise materials of one or more of the IUPAC Groups 4, 6, 11, 12, 13, 14, and 15 elements, plastic material, silicon dioxide, glass, fused silica, mica, ceramic, or metals deposited on the aforementioned substrates. Exemplary substrates include, but are not limited to, glass, modified and/or functionalized glass, hydrogels, films, membranes, plastics (including e.g., acrylics, polystyrene, copolymers of styrene and other materials, polypropylene, polyethylene, polybutylene, polyurethanes, Teflon^{™}, cyclic olefins, polyimides etc.), nylon, ceramics, resins, Zeonor, silica or silica-based materials including silicon and modified silicon, carbon, quartz, metals, inorganic glasses, optical fiber bundles, and polymers, such as polystyrene, cyclic olefin copolymers (COCs), cyclic olefin polymers (COPs), polypropylene, polyethylene and polycarbonate. In some embodiments, the substrate is a glass substrate.

A substrate can be of any desired shape. For example, a substrate can be typically a thin (e.g., sub-centimeter), flat shape (e.g., square, rectangle or a circle). In some embodiments, a substrate structure has rounded corners (e.g., for increased safety or robustness). In some embodiments, a substrate structure has one or more cut-off corners (e.g., for use with a slide clamp or cross-table). In some embodiments, where a substrate structure is flat, the substrate structure can be any appropriate type of support having a flat surface (e.g., a chip, wafer, die, or a slide such as a microscope slide).

In some embodiments, the surface of the substrate is coated. In some embodiments, the surface of the substrate is coated with a photoresist. In some embodiments, the method described herein comprises applying the photoresist to the substrate. In some embodiments, the substrate comprises a pattern of oligonucleotide molecules on the substrate prior to photoresist(s) being applied to the substrate. In some embodiments, the substrate does not comprise a pattern of oligonucleotide molecules on the substrate prior to photoresist(s) being applied to the substrate. In some embodiments where the substrate does not comprise oligonucleotide molecules prior to the application of photoresist(s), the substrate comprises a plurality of functional groups. In some embodiments, the plurality of functional groups of the substrate are not protected, for example, by photo-sensitive groups, moieties, or molecules. In some embodiments, the plurality of functional groups are aldehyde groups. In some embodiments, the plurality of functional groups of the substrate are click chemistry groups. The click chemistry group may be capable of various chemical reactions, which include but are not limited to, a nucleophilic addition reaction, a cyclopropane-tetrazine reaction, a strain-promoted azide-alkyne cycloaddition (SPAAC) reaction, an alkyne hydrothiolation reaction, an alkene hydrothiolation reaction, a strain-promoted alkyne-nitrone cycloaddition (SPANC) reaction, an inverse electron-demand Diels-Alder (IED-DA) reaction, a cyanobenzothiazole condensation reaction, an aldehyde/ketone condensation reaction, or a Cu(I)-catalyzed azide-alkyne cycloaddition (CuAAC) reaction. In some embodiments, the plurality of functional groups on the substrate react with functional groups in functionalized oligonucleotide molecules. In some embodiments, the functional groups in the functionalized oligonucleotide molecules are amino groups. In some embodiments, the functionalized oligonucleotide molecules are 5' amineterminated.

In some embodiments, during the contact between the functionalized oligonucleotide molecules and the functional groups on the substrate, the substrate is heated to dryness. In some embodiments, after the contact between the functionalized oligonucleotide molecules and the functional groups on the substrate, the substrate is heated to dryness. In some embodiments according to any one of the methods described herein, the method further comprises blocking unreacted functional groups of the substrate. In some embodiments, the method comprises rendering the reaction between functional groups of the substrate and the functionalized oligonucleotide molecules irreversible. For example, the aldehyde groups of the substrate are reacted with 5' amino groups of the functionalized oligonucleotide molecules, and the substrate is contacted with a reagent to block unreacted aldehyde groups and render the reaction irreversible. In some embodiment, the reagent is a reductive agent. In some embodiment, the reagent is sodium borohydride.

### A. PHOTORESIST

In some embodiments, before or after blocking or ablating oligonucleotide molecules in a boundary region on the substrate, a method disclosed herein comprises (a) irradiating a substrate comprising an unmasked first one or more spot regions and a masked second one or more spot regions, whereby a photoresist in the first region is degraded to render oligonucleotide molecules in the first one or more spot regions available for hybridization and/or ligation, whereas oligonucleotide molecules in the second region one or more spot regions are protected by a photoresist in the second region from hybridization and/or ligation; and (b) attaching an oligonucleotide comprising a barcode sequence to oligonucleotide molecules in the first one or more spot regions via hybridization and/or ligation, wherein oligonucleotide molecules in the second one or more spot regions do not receive the barcode sequence, thereby providing on the substrate an array comprising different oligonucleotide molecules in the first and second regions.

In some embodiments, oligonucleotide molecules on the substrate comprise one or more common sequences. In some embodiments, oligonucleotide molecules on the substrate comprise functional groups. In some embodiments, the functional groups are not protected by a photo-sensitive moiety prior to the irradiating step. In some embodiments, the functional groups are 3' hydroxyl groups of nucleotides. In some embodiments, the method further comprises forming a pattern of oligonucleotide molecules on the substrate prior to applying the photoresist to the substrate. In some embodiments, forming the pattern of oligonucleotide molecules comprises: irradiating a substrate comprising a plurality of functional groups and a photoresist through a patterned mask, whereby the photoresist in a first region of the substrate is degraded, rendering functional groups in the first region available for reacting with functional groups in functionalized oligonucleotide molecules, whereas functional groups in a second region of the substrate are protected by the photoresist from reacting with functional groups in the oligonucleotide molecules; and contacting the substrate with the functionalized oligonucleotide molecules, wherein the functionalized oligonucleotide molecules are coupled to functional groups in the first region but not to functional groups in the second region, thereby forming a pattern of oligonucleotide molecules on the substrate. In some embodiments, the functional groups in the functionalized oligonucleotide molecules are amino groups. In some embodiments, the method further comprises rendering the reaction between functional groups of the substrate and the functionalized oligonucleotide molecules irreversible. In some embodiments, the irradiating and contacting steps are repeated in one or more cycles. In some embodiments, the photoresist is not removed prior to, during, or between the one or more cycles. In some embodiments, the substrate is irradiated through a patterned mask. In some embodiments, the method comprises removing the patterned mask after the irradiating step, wherein the same patterned mask is re-used in a subsequent cycle of the irradiating and contacting steps. In some embodiments, the photoresist in the first region of the substrate is dissolved by a developer and removed. In some embodiments, the barcode sequence is between about 4 and about 25 nucleotides in length. In some embodiments, the oligonucleotide comprising the barcode sequence is between about 10 and about 50 nucleotides in length. In some embodiments, the oligonucleotide comprising the barcode sequence is hybridized to an oligonucleotide molecule in the first region. In some embodiments, the oligonucleotide comprising the barcode sequence is ligated to an oligonucleotide molecule in the first region. In some embodiments, the oligonucleotide comprising the barcode sequence is hybridized to a splint which is in turn hybridized to an oligonucleotide molecule in the first region. In some embodiments, the method further comprises ligating the oligonucleotide comprising the barcode sequence to the oligonucleotide molecule to generate a barcoded oligonucleotide molecule in the first region. In some embodiments, the method further comprises blocking the 3' or 5' termini of barcoded oligonucleotide molecules and/or unligated oligonucleotide molecules in the first region from ligation. In some embodiments, the photoresist is not removed prior to, during, or between any of the cycles. In some embodiments, the feature is no more than 10 microns in diameter.

In some embodiments, a method disclosed herein comprises (a) irradiating a substrate comprising an unmasked first region and a masked second region, whereby a photoresist in the first region is degraded to render oligonucleotide molecules in the first region available for hybridization and/or ligation, whereas oligonucleotide molecules in the second region are protected by the photoresist in the second region from hybridization and/or ligation; and (b) contacting oligonucleotide molecules in the first region with a first splint and a first oligonucleotide comprising a first barcode sequence, wherein the first splint hybridizes to the first oligonucleotide and the oligonucleotide molecules in the first region, wherein the first oligonucleotide is ligated to the oligonucleotide molecules in the first region, and the first oligonucleotide is not ligated to oligonucleotide molecules in the second region, thereby providing on the substrate an array comprising different oligonucleotide molecules in the first and second regions. In some embodiments, the photoresist is a first photoresist, and the first oligonucleotide is ligated to the oligonucleotide molecules in the first region to generate first extended oligonucleotide molecules, and the method further comprises: (c) applying a second photoresist to the substrate, optionally wherein the second photoresist is applied after the first photoresist is removed from the substrate; (d) irradiating the substrate while the first region is masked and the second region is unmasked, whereby the first or second photoresist in the second region is degraded to render oligonucleotide molecules in the second region available for hybridization and/or ligation, whereas the first extended oligonucleotide molecules in the first region are protected by the second photoresist in the first region from hybridization and/or ligation; and (e) contacting oligonucleotide molecules in the second region with a second splint and a second oligonucleotide comprising a second barcode sequence, wherein the second splint hybridizes to the second oligonucleotide and the oligonucleotide molecules in the second region, wherein the second oligonucleotide is ligated to the oligonucleotide molecules in the second region to generate second extended oligonucleotide molecules, and the second oligonucleotide is not ligated to the first extended oligonucleotide molecules in the first region.

In some embodiments, the oligonucleotide molecules are protected from hybridization by a photoresist covering the oligonucleotide molecules. In some embodiments, the oligonucleotide molecules are protected from ligation by a photoresist covering the oligonucleotide molecules. In some embodiments, the oligonucleotide molecules are protected from hybridization and ligation by a photoresist covering the oligonucleotide molecules. In some embodiments, the photoresist in irradiated regions is removed. In some embodiments, the photoresist in masked or non-irradiated regions is not removed.

A photoresist is a light-sensitive material used in processes (such as photolithography and photoengraving) to form a pattern on a surface. A photoresist may comprise a polymer, a sensitizer, and/or a solvent. The photoresist composition used herein is not limited to any specific proportions of the various components. Photoresists can be classified as positive or negative. In positive photoresists, the photochemical reaction that occurs during light exposure weakens the polymer, making it more soluble to developer, so a positive pattern is achieved. In the case of negative photoresists, exposure to light causes polymerization of the photoresist, and therefore the negative photoresist remains on the surface of the substrate where it is exposed, and the developer solution removes only the unexposed areas. In some embodiments, the photoresist used herein is a negative photoresist. In some embodiments, the photoresist used herein is a positive photoresist. In some embodiments, the photoresist is removable with UV light.

The photoresist may experience changes in pH upon irradiation. In some embodiments, the photoresist in one or more regions comprises a photoacid generator (PAG). In some embodiments, the photoresist in one or more other regions comprises a PAG. In some embodiments, the photoresist in the one or more regions and the one or more other regions comprises a PAG. In some embodiments, the photoresist in the one or more regions and the one or more other regions comprises the same PAG. In some embodiments, the photoresist in the one or more regions and the one or more other regions comprises different PAG. In some embodiments, the PAG or PAGs irreversibly release protons upon absorption of light. PAGs may be used as components of photocurable polymer formulations and chemically amplified photoresists. Examples of PAGs include triphenylsulfonium triflate, diphenylsulfonium triflate, diphenyliodonium nitrate, N-Hydroxynaphthalimide triflate, triarylsulfonium hexafluorophosphate salts, N-hydroxy-5-norbornene-2,3-dicarboximide perfluoro-1-butanesulfonate, bis(4-tert-butylphenyl)iodonium perfluoro-1-butanesulfonate, etc.

In some embodiments, the photoresist further comprises an acid scavenger. In some embodiments, the photoresist in the one or more regions and the one or more other regions comprises the same acid scavenger. In some embodiments, the photoresist in the one or more regions and the one or more other regions comprises different acid scavengers. In some embodiments, an acid scavenger acts to neutralize, adsorb and/or buffer acids, and may comprise a base or alkaline compound. In some embodiments, acid scavengers act to reduce the amount or concentration of protons or protonated water. In some embodiments, an acid scavenger acts to neutralize, diminish, or buffer acid produced by a PAG. In some embodiments, an acid scavenger exhibits little or no stratification over time or following exposure to heat. In some embodiments, acid scavengers may be further subdivided into "organic bases" and "polymeric bases." A polymeric base is an acid scavenger (e.g., basic unit) attached to a longer polymeric unit. A polymer is typically composed of a number of coupled or linked monomers. The monomers can be the same (to form a homopolymer) or different (to form a copolymer). In a polymeric base, at least some of the monomers act as acid scavengers. An organic base is a base which is joined to or part of a non-polymeric unit. Non-limiting examples of organic bases include, without limitation, amine compounds (e.g., primary, secondary and tertiary amines). Generally any type of acid scavenger, defined here as a traditional Lewis Base, an electron pair donor, can be used in accordance with the present disclosure.

In some embodiments, the photoresist further comprises a base quencher. Base quenchers may be used in photoresist formulations to improve performance by quenching reactions of photoacids that diffuse into unexposed regions. Base quenchers may comprise aliphatic amines, aromatic amines, carboxylates, hydroxides, or combinations thereof. Examples of base quenchers include but are not limited to, trioctylamine, 1,8-diazabicyclo[5.4.0]undec-7-ene (DBU), 1-piperidineethanol (1PE), tetrabutylammonium hydroxide (TBAH), dimethylamino pyridine, 7-diethylamino-4-methyl coumarin (Coumarin 1), tertiary amines, sterically hindered diamine and guanidine bases such as 1,8-bis(dimethylamino)naphthalene (PROTON SPONGE), berberine, or polymeric amines such as in the PLURONIC or TETRONIC series commercially available from BASF. In some embodiments, the photoresist in the one or more regions and the one or more other regions comprises the same base quencher. In some embodiments, the photoresist in the one or more regions and the one or more other regions comprises different base quenchers.

In some embodiments, the photoresist further comprises a photosensitizer. A photosensitizer is a molecule that produces a chemical change in another molecule in a photochemical process. Photosensitizers are commonly used in polymer chemistry in reactions such as photopolymerization, photocrosslinking, and photodegradation. Photosensitizers generally act by absorbing ultraviolet or visible region of electromagnetic radiation and transferring it to adjacent molecules. In some embodiments, photosensitizer shifts the photo sensitivity to a longer wavelength of electromagnetic radiation. The sensitizer, also called a photosensitizer, is capable of activating the PAG at, for example, a longer wavelength of light in accordance with an aspect of the present invention. Preferably, the concentration of the sensitizer is greater than that of the PAG, such as 1.1 times to 5 times greater, for example, 1.1 times to 3 times greater the concentration of PAG. Exemplary sensitizers suitable for use in the invention include but are not limited to, isopropylthioxanthone (ITX) and 10H-phenoxazine (PhX). In some embodiments, the photoresist in the one or more regions and the one or more other regions comprises the same photosensitizer. In some embodiments, the photoresist in the one or more regions and the one or more other regions comprises different photosensitizers.

In some embodiments, the photoresist further comprises a matrix. The matrix generally refers to polymeric materials that may provide sufficient adhesion to the substrate when the photoresist formulation is applied to the top surface of the substrate, and may form a substantially uniform film when dissolved in a solvent and spread on top of a substrate. Examples of a matrix may include, but are not limited to, polyester, polyimide, polyethylene naphthalate (PEN), polyvinyl chloride (PVC), polymethylmethacrylate (PMMA) and polycarbonate, or a combination thereof. The matrix may be chosen based on the wavelength of the radiation used for the generation of acid when using the photoresist formulation, the adhesion properties of the matrix to the top surface of the substrate, the compatibility of the matrix to other components of the formulation, and the ease of removable or degradation (if needed) after use. In some embodiments, the photoresist in the one or more regions and the one or more other regions comprises the same matrix. In some embodiments, the photoresist in the one or more regions and the one or more other regions comprises different matrices.

In some embodiments, the photoresist further comprises a surfactant. Surfactants may be used to improve coating uniformity, and may include ionic, non-ionic, monomeric, oligomeric, and polymeric species, or combinations thereof. Examples of possible surfactants include fluorine-containing surfactants such as the FLUORAD series available from 3M Company in St. Paul, Minn., and siloxane-containing surfactants such as the SILWET series available from Union Carbide Corporation in Danbury, Conn. In some embodiments, the photoresist in the one or more regions and the one or more other regions comprises the same surfactant. In some embodiments, the photoresist in the one or more regions and the one or more other regions comprises different surfactants.

In some embodiments, the photoresist further comprises a casting solvent. A casting solvent may be used so that the photoresist may be applied evenly on the substrate surface to provide a defect-free coating. Examples of suitable casting solvents may include ethers, glycol ethers, aromatic hydrocarbons, ketones, esters, ethyl lactate, γ-butyrolactone, cyclohexanone, ethoxyethylpropionate (EEP), a combination of EEP and gamma-butyrolactone (GBL), and propylene glycol methyl ether acetate (PGMEA). In some embodiments, the photoresist in the one or more regions and the one or more other regions comprises the same casting solvent. In some embodiments, the photoresist in the one or more regions and the one or more other regions comprises different casting solvents.

Methods of applying photoresist to the substrate include, but are not limited to, dipping, spreading, spraying, or any combination thereof. In some embodiments, the photoresist is applied via spin coating, thereby forming a photoresist layer on the substrate.

In some embodiments, the photoresist is in direct contact with the oligonucleotides on the substrate. In some embodiments, the oligonucleotide molecules on the substrate are embedded in the photoresist. In some embodiments, the photoresist is not in direct contact with the oligonucleotides. In some embodiments, oligonucleotide molecules on the substrate are embedded in an underlayer that is underneath the photoresist. For example, oligonucleotide molecules on the substrate nay be embedded in a soluble polymer underlayer (e.g., a soluble polyimide underlayer (XU-218)), and the photoresist forms a photoresist layer on top of the underlayer.

In some embodiments, the photoresist may be removed and re-applied. For example, the photoresist may be stripped from the substrate and/or the oligonucleotides ligated to the substrate. Removal of photoresist can be accomplished with various degrees of effectiveness. In some embodiments, the photoresist is completely removed from the substrate and/or the oligonucleotides ligated to the substrate before re-application. Methods of removing photoresist may include, but are not limited to, using organic solvent mixtures, using liquid chemicals, exposure to a plasma environment, or other dry techniques such as UV/O₃ exposure. In some embodiments, the photoresist is stripped using organic solvent.

In some embodiments, one or more photomasks may be used to selectively remove photoresist on the substrate. The mask is designed in such a way that the exposure sites can be selected, and thus specify the coordinates on the array where each nucleotide can be attached. The process can be repeated, a new mask is applied activating different sets of sites and coupling different bases, allowing arbitrary oligonucleotides to be constructed at each site. This process can be used to synthesize hundreds of thousands or millions of different oligonucleotides. In some embodiments, the substrate is irradiated through a patterned mask. The mask may be an opaque plate or film with transparent areas that allow light to shine through in a pre-defined pattern. After the irradiation step, the mask may be removed, translated to a different region on the substrate, or rotated. In some embodiments, a different photomasking pattern may be used in each barcoding round. In some embodiments, the same photomasking pattern may be used in each barcoding round. Using a series of photomasks, photoresist in desired regions of the substrate may be iteratively irradiated and subsequently removed.

The material of the photomask used herein may comprise silica with chrome in the opaque part. For example, the photomask may be transparent fused silica blanks covered with a pattern defined with a chrome metal absorbing film. The photomask may be used at various irradiation wavelengths, which include but are not limited to, 365 nm, 248 nm, and 193 nm.

### B. POLYMERS

In some embodiments, before or after blocking or ablating oligonucleotide molecules in a boundary region on the substrate, a method disclosed herein comprises irradiating a first polynucleotide immobilized in a spot region on a substrate with a first light while a second polynucleotide immobilized in another spot region on the substrate is not irradiated with the first light, wherein the first polynucleotide is bound to a first photo-cleavable polymer that inhibits or blocks hybridization and/or ligation to the first polynucleotide, and the second polynucleotide is bound to a second photo-cleavable polymer that inhibits or blocks hybridization and/or ligation to the second polynucleotide, thereby cleaving the first photocleavable polymer such that the inhibition or blocking of hybridization and/or ligation to the first polynucleotide is reduced or eliminated, whereas hybridization and/or ligation to the second polynucleotide remains inhibited or blocked by the second photo-cleavable polymer, wherein a first barcode is attached to the first polynucleotide via hybridization and/or ligation, thereby providing on the substrate an array comprising the first and second polynucleotides, wherein the first polynucleotide is barcoded with the first barcode and the second polynucleotide is not barcoded with the first barcode.

In some embodiments, the method further comprises irradiating the second polynucleotide with a second light, thereby cleaving the second photo-cleavable polymer such that the inhibition or blocking of hybridization and/or ligation to the second polynucleotide is reduced or eliminated. In some embodiments, the second polynucleotide is irradiated with the second light while the first polynucleotide is not irradiated with the second light. In some embodiments, the method further comprises attaching a second barcode to the second polynucleotide via hybridization and/or ligation, thereby providing on the substrate an array comprising the first polynucleotide barcoded with the first barcode and the second polynucleotide barcoded with the second barcode. In some embodiments, hybridization and/or ligation to the first polynucleotide barcoded with the first barcode is inhibited or blocked. In some embodiments, the first barcode comprises a first photo-cleavable moiety that inhibits or blocks hybridization and/or ligation, thereby inhibiting or blocking hybridization and/or ligation to the first polynucleotide barcoded with the first barcode. In some embodiments, the first photo-cleavable moiety comprises a photo-caged nucleobase, a photo-cleavable linker, a photocleavable hairpin and/or a photo-caged 3'-hydroxyl group. In some embodiments, the first barcode is a DNA oligonucleotide. In some embodiments, the first barcode is between about 5 and about 20 nucleotides in length. In some embodiments, the substrate comprises a plurality of differentially barcoded polynucleotides immobilized thereon. In some embodiments, the irradiating comprises using a photomask to selectively irradiate the first polynucleotide or the second polynucleotide. In some embodiments, the attachment of the first barcode and/or the second barcode comprises ligating one end of the first/second barcode to one end of the first/second polynucleotide, respectively. In some embodiments, the attachment of the first barcode comprises hybridizing one end of the first barcode and one end of the first polynucleotide to a first splint. In some embodiments, the method further comprises ligating the first barcode to the first polynucleotide hybridized to the first splint. In some embodiments, the first barcode is directly ligated to the first polynucleotide, without gap filling. In some embodiments, ligating the first barcode to the first polynucleotide is preceded by gap filling. In some embodiments, the first splint is a DNA oligonucleotides at least 4 nucleotides in length. In some embodiments, the first photo-cleavable polymer and/or the second photo-cleavable polymer are UV degradable. In some embodiments, the first photo-cleavable polymer and/or the second photo-cleavable polymer comprise a polyethylenimine (PEI).

In some embodiments, a method disclosed herein comprises: (a1) irradiating polynucleotide P1 immobilized on a substrate with light while polynucleotide P2 immobilized on the substrate is photomasked, wherein polynucleotides P1 and P2 are bound to a photocleavable polymer that inhibits or blocks hybridization and/or ligation to P1 and P2, respectively, thereby cleaving the photo-cleavable polymer to allow hybridization and/or ligation to P1, whereas hybridization and/or ligation to P2 remain inhibited or blocked by the photo-cleavable polymer; and (b 1) attaching barcode 1A to P1 via hybridization and/or ligation to form a barcoded polynucleotide 1A-P1, thereby providing on the substrate an array comprising polynucleotides 1A-P1 and P2. In some embodiments, the method further comprises: (c1) irradiating P2 with light, thereby cleaving the photo-cleavable polymer to allow hybridization and/or ligation to P2; and (d1) attaching barcode 1B to P2 via hybridization and/or ligation to form a barcoded polynucleotide 1B-P2, thereby providing on the substrate an array comprising barcoded polynucleotides 1A-P1 and 1B-P2. In some embodiments, polynucleotides of different nucleic acid sequences are immobilized on the substrate in a pattern comprising rows and columns prior to the irradiation.

In some embodiments, the oligonucleotide molecules are protected from hybridization by a polymer binding to the oligonucleotide molecules. In some embodiments, the oligonucleotide molecules are protected from ligation by a polymer binding to the oligonucleotide molecules. In some embodiments, the oligonucleotide molecules are protected from hybridization and ligation by a polymer binding to the oligonucleotide molecules. In some embodiments, the polymer is a photo-cleavable polymer. In some embodiments, the polymer (e.g., photo-cleavable polymer) binds to the oligonucleotide molecules in a non-sequence specific manner. In some embodiments, the photo-cleavable polymers in irradiated regions are cleaved and the photo-cleavable polymers in masked or non-irradiated regions are not cleaved.

In some embodiments, a photo-cleavable polymer disclosed herein is not part of an oligonucleotide. In some embodiments, a photo-cleavable polymer disclosed herein is not covalently bonded to an oligonucleotide. In some embodiments, a photo-cleavable polymer disclosed herein is noncovalently bound to an oligonucleotide. In some embodiments, the oligonucleotide is prevented from hybridization to a nucleic acid such as a splint. In some embodiments, a photo-cleavable polymer disclosed herein inhibits or blocks ligation to either end of the oligonucleotide, while hybridization of a nucleic acid to the oligonucleotide may or may not be inhibited or blocked. For example, the photo-cleavable polymer bound to an oligonucleotide may inhibit or block the 3' or 5' end of the oligonucleotide from chemical or enzymatic ligation, e.g., even when a splint may hybridize to the oligonucleotide in order to bring a ligation partner in proximity to the 3' or 5' end of the oligonucleotide. In some embodiments, the photo-cleavable polymer may cap the 3' or 5' end of the oligonucleotide.

In some embodiments, the photo-cleavable polymer is UV degradable. In some embodiments, the photo-cleavable polymer comprises a UV-degradable group (e.g., a UV-degradable functional moiety). In some embodiments, the UV-degradable group is within the backbone or at each subunit of the photo-cleavable polymer. In some embodiments, the UV-degradable group comprises a nitrobenzyl group, e.g., within a PEG (polyethylene glycol), a PDMS (polydimethylsiloxane), or a polyethylenimine (PEI), for example, in the polymer backbone or at each subunit. Complete cleavage of the nitrobenzyl group(s) is not required for nucleic acid release. In some embodiments, cleavage of a portion of the UV-degradable groups is sufficient to render the oligonucleotides available for hybridization and/or ligation.

In some embodiments, the photo-cleavable polymer is synthetic, semisynthetic, or natural. In some embodiments, the photo-cleavable polymer comprises a material selected from the group consisting of a PEG (polyethylene glycol), a PDMS (polydimethylsiloxane), a polyethylenimine (PEI), a polyacrylate, a lipid, a nanoparticle, a DNA, an RNA, a synthetic oligodeoxynucleotide (ODN), a xeno nucleic acid (XNA), a peptide nucleic acid (PNA), a locked nucleic acid (LNA), a 1,5-anhydrohexitol nucleic acid (HNA), a cyclohexene nucleic acid (CeNA), a threose nucleic acid (TNA), a glycol nucleic acid (GNA), a fluoro arabino nucleic acid (FANA), and a polypeptide. In some embodiments, the polyacrylate and/or the lipid is cationic, optionally wherein the cationic lipid is Lipofectamine. In some embodiments, the photo-cleavable polymer comprises the formulaa of (dNTP)₆-PC-(dNTP)₆-PC-(dNTP)₆-PC-(dNTP)₆, wherein PC is a photo-cleavable moiety.

### C. OLIGONUCLEOTIDES COMPRISING PHOTO-CLEAVABLE MOIETIES

In some embodiments, before or after blocking or ablating oligonucleotide molecules in a boundary region on the substrate, a method disclosed herein comprises irradiating a first polynucleotide immobilized in a spot region on a substrate with a first light while a second polynucleotide immobilized in another spot region on the substrate is not irradiated with the first light, wherein the first polynucleotide comprises a first photo-cleavable moiety that inhibits or blocks hybridization and/or ligation to the first polynucleotide, and the second polynucleotide comprises a second photo-cleavable moiety that inhibits or blocks hybridization and/or ligation to the second polynucleotide, thereby cleaving the first photo-cleavable moiety such that the inhibition or blocking of hybridization and/or ligation to the first polynucleotide is reduced or eliminated, whereas hybridization and/or ligation to the second polynucleotide remain inhibited or blocked by the second photo-cleavable moiety, wherein a first barcode is attached to the first polynucleotide via hybridization and/or ligation, thereby providing on the substrate an array comprising the first and second polynucleotides, wherein the first polynucleotide is barcoded with the first barcode and the second polynucleotide is not barcoded with the first barcode. In some embodiments, blocking the oligonucleotide molecules in the boundary region comprises adding a blocking group (e.g., adding a 3' dideoxy, a non-ligating 3' phosphoramidate, or a triphenylmethyl (trityl) group) to the oligonucleotide molecules in the boundary region, wherein the blocking group is not photo-cleavable.

In some embodiments, the method further comprises irradiating the second polynucleotide with a second light, thereby cleaving the second photo-cleavable moiety such that the inhibition or blocking of hybridization and/or ligation to the second polynucleotide is reduced or eliminated. In some embodiments, the second polynucleotide is irradiated with the second light while the first polynucleotide is not irradiated with the second light. In some embodiments, the method further comprises attaching a second barcode to the second polynucleotide via hybridization and/or ligation, thereby providing on the substrate an array comprising the first polynucleotide barcoded with the first barcode and the second polynucleotide barcoded with the second barcode. In some embodiments, hybridization and/or ligation to the first polynucleotide barcoded with the first barcode is inhibited or blocked, and/or hybridization and/or ligation to the second polynucleotide barcoded with the second barcode is inhibited or blocked. In some embodiments, the first barcode comprises a third photo-cleavable moiety that inhibits or blocks hybridization and/or ligation, thereby inhibiting or blocking hybridization and/or ligation to the first polynucleotide barcoded with the first barcode. In some embodiments, the first photo-cleavable moiety and/or the second photo-cleavable moiety comprise a photo-caged nucleobase. In some embodiments, the first photo-cleavable moiety and/or the second photo-cleavable moiety comprise a photo-cleavable hairpin. In some embodiments, the first photo-cleavable moiety and/or the second photo-cleavable moiety comprise a photo-caged 3'-hydroxyl group. In some embodiments, the substrate comprises a plurality of differentially barcoded polynucleotides immobilized thereon. In some embodiments, irradiating the sample comprises using a photomask to selectively irradiate the first polynucleotide or the second polynucleotide. In some embodiments, the attachment of the first barcode-comprises ligating one end of the first barcode to one end of the first polynucleotide. In some embodiments, the attachment of the first barcode comprises hybridizing one end of the first barcode and one end of the first polynucleotide to a first splint. In some embodiments, the method further comprises ligating the first barcode to the first polynucleotide hybridized to the first splint. In some embodiments, ligating the first barcode to the first polynucleotide, respectively, is preceded by gap filling.

In some embodiments, a method disclosed herein comprises (a1) irradiating polynucleotide P1 immobilized on a substrate with light while polynucleotide P2 immobilized on the substrate is photomasked, wherein polynucleotides P1 and P2 comprise a photo-cleavable moiety that inhibits or blocks hybridization and/or ligation to P1 and P2, respectively, thereby cleaving the photo-cleavable moiety to allow hybridization and/or ligation to P1, whereas hybridization and/or ligation to P2 remain inhibited or blocked by the photo-cleavable moiety; and (b1) attaching barcode 1A to P1 via hybridization and/or ligation to form a barcoded polynucleotide 1A-P1, wherein barcode 1A comprises the photo-cleavable moiety which inhibits or blocks hybridization and/or ligation to 1A-P1, thereby providing on the substrate an array comprising polynucleotides 1A-P1 and P2 each comprising the photo-cleavable moiety that inhibits or blocks hybridization and/or ligation. In some embodiments, the method further comprises: (c1) irradiating P2 with light while 1A-P1 is photomasked, thereby cleaving the photo-cleavable moiety to allow hybridization and/or ligation to P2, whereas hybridization and/or ligation to 1A-P1 remain inhibited or blocked by the photo-cleavable moiety; and (d1) attaching barcode 1B to P2 via hybridization and/or ligation to form a barcoded polynucleotide 1B-P2, wherein barcode 1B comprises the photo-cleavable moiety which inhibits or blocks hybridization and/or ligation to 1B-P2, thereby providing on the substrate an array comprising barcoded polynucleotides 1A-P1 and 1B-P2 each comprising the photo-cleavable moiety that inhibits or blocks hybridization and/or ligation. In some embodiments, the method further comprises: (a2) irradiating one of 1A-P1 and 1B-P2 with light while the other is photomasked, thereby cleaving the photo-cleavable moiety to allow hybridization and/or ligation to the irradiated polynucleotide, whereas hybridization and/or ligation to the photomasked polynucleotide remains inhibited or blocked by the photo-cleavable moiety; and (b2) attaching barcode 2A to the irradiated polynucleotide via hybridization and/or ligation to form a 2A-barcoded polynucleotide, wherein barcode 2A comprises the photo-cleavable moiety which inhibits or blocks hybridization and/or ligation, thereby providing on the substrate an array comprising barcoded polynucleotides each comprising the photo-cleavable moiety that inhibits or blocks hybridization and/or ligation. In some embodiments, the method further comprises (c2) irradiating the photomasked polynucleotide in step a2 with light while the 2A-barcoded polynucleotide is photomasked, thereby cleaving the photo-cleavable moiety to allow hybridization and/or ligation, whereas hybridization and/or ligation to the 2A-barcoded polynucleotide remain inhibited or blocked by the photo-cleavable moiety; and (d2) attaching barcode 2B to the irradiated polynucleotide in step c2 via hybridization and/or ligation to form a 2B-barcoded polynucleotide, wherein barcode 2B comprises the photo-cleavable moiety which inhibits or blocks hybridization and/or ligation, thereby providing on the substrate an array comprising barcoded polynucleotides each comprising the photo-cleavable moiety that inhibits or blocks hybridization and/or ligation. In some embodiments, steps al-dl form round 1 and steps a2-d2 form round 2, the method further comprising steps a*i*-d*i* in round *i*, wherein barcodes *i*A and *i*B are attached to provide barcoded polynucleotides on the substrate, and wherein *i* is an integer greater than 2.

In some embodiments, the photo-cleavable moiety comprises a photo-caged nucleobase. In some embodiments, the photo-caged nucleobase is a photo-caged deoxythymidine (dT). In some embodiments, the photo-cleavable moiety comprises the following structure: 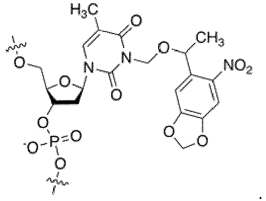 In some embodiments, the photo-cleavable moiety comprises a photo-cleavable hairpin. In some embodiments, the photo-cleavable moiety comprises the following structure: 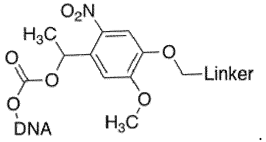 In some embodiments, the photocleavable moiety comprises a photo-caged 3'-hydroxyl group. In some embodiments, the photocleavable moiety comprises the following structure: 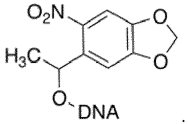 In some embodiments, polynucleotides of different nucleic acid sequences are immobilized on the substrate in a pattern comprising rows and columns prior to the irradiation.

In some embodiments, the oligonucleotide molecules are protected from hybridization by a protective group of each oligonucleotide molecule. In some embodiments, the oligonucleotide molecules are protected from ligation by a protective group of each oligonucleotide molecule. In some embodiments, the oligonucleotide molecules are protected from hybridization and ligation by a protective group of each oligonucleotide molecule. In some embodiments, the protective group is a photo-cleavable protective group. In some embodiments, the photo-cleavable protective groups in irradiated regions are cleaved and the photo-cleavable protective groups in masked or non-irradiated regions are not cleaved. Specifically, in some embodiments, the oligonucleotide molecules are protected from hybridization using photo-caged oligonucleotides. In some embodiments, the oligonucleotide molecules are protected from ligation using photo-caged oligonucleotides. In some embodiments, the oligonucleotide molecules are protected from hybridization and ligation using photo-caged oligonucleotides. In some embodiments, the photo-caged oligonucleotides comprises one or more photo-cleavable moieties, such as a photo-cleavable protective group. For example, hybridization can be blocked using a synthetic nucleotide with a photo-cleavable protecting group on a nucleobase and/or a photo-cleavable hairpin that dissociates upon cleavage. In other examples, ligation can be controlled using a photo-cleavable moiety, such as a photo-caged 3'-hydroxyl group.

In some embodiments, a photo-cleavable moiety disclosed herein is part of an oligonucleotide (e.g., a first oligonucleotide or a second oligonucleotide), such as oligonucleotide molecules in one or more other regions on the substrate, and inhibits or blocks hybridization to the oligonucleotide (e.g., the hybridization of a splint and/or a third oligonucleotide to the first and/or second oligonucleotide), but does not inhibit or block hybridization to the oligonucleotide molecules in one or more regions on the substrate.. In some embodiments, the oligonucleotide is prevented from hybridization to a nucleic acid such as a splint. In some embodiments, a photo-cleavable moiety disclosed herein is part of an oligonucleotide and inhibits or blocks ligation to either end of the oligonucleotide, while hybridization of a nucleic acid to the oligonucleotide may or may not be inhibited or blocked. For example, the photo-cleavable moiety may inhibit or block the 3' or 5' end of the oligonucleotide from chemical or enzymatic ligation, *e.g*., even when a splint may hybridize to the oligonucleotide in order to bring a ligation partner in proximity to the 3' or 5' end of the oligonucleotide. In some embodiments, the photo-cleavable moiety may cap the 3' or 5' end of the oligonucleotide.

In some embodiments, the irradiation results in cleavage of the photo-cleavable moiety such that the inhibition or blocking of hybridization and/or ligation to the oligonucleotide molecules in the one or more regions is reduced or eliminated, whereas hybridization and/or ligation to the oligonucleotide nucleotide molecules in one or more other regions remain inhibited or blocked by a second photo-cleavable moiety.

In any of the preceding embodiments, physical masks, e.g., a photolithography mask which is an opaque plate or film with transparent areas that allow light to shine through in a defined pattern, may be used. In any of the preceding embodiments, different protection groups and/or photolabile groups may be used. In any of the preceding embodiments, the light can have a wavelength between about 365 nm and about 440 nm, for example, about 366 nm, 405 nm, or 436 nm.

### V. COMPOSITIONS, KITS, AND METHODS OF USE

Also provided are compositions produced according to the methods described herein. These compositions include nucleic acid molecules and complexes, such as hybridization complexes, and kits and articles of manufacture (such as arrays) comprising such molecules and complexes.

In particular embodiments, provided herein is a composition comprising: a substrate comprising a plurality of spot regions and a boundary region between the plurality of spot regions, wherein the boundary region comprises blocked oligonucleotide molecules immobilized in the boundary region and oligonucleotide molecules in the spot regions are not blocked. In some embodiments, the composition further comprises a photoresist covering the spot regions.

Also provided herein are arrays comprising any one or more of the molecules, complexes, and/or compositions disclosed herein. Typically, an array includes at least two distinct nucleic acid polymers that differ by monomeric sequence immobilized on, e.g., covalently to, different and known locations on the substrate surface. In certain embodiments, each distinct nucleic acid sequence of the array is typically present as a composition of multiple copies of the polymer on the substrate surface, *e.g.* as a spot on the surface of the substrate. The number of distinct nucleic acid sequences, and hence spots or similar structures, present on the array may vary, but is generally at least, usually at least 5 and more usually at least 10, where the number of different spots on the array may be as a high as 50, 100, 500, 1000, 10,000, 1,000,000, 10,000,000 or higher, depending on the intended use of the array. The spots of distinct polymers present on the array surface are generally present as a pattern, where the pattern may be in the form of organized rows and columns of spots, *e.g.* a grid of spots, across the substrate surface, a series of curvilinear rows across the substrate surface, *e.g.* a series of concentric circles or semi-circles of spots, and the like. The density of spots present on the array surface may vary, but is generally at least about 10 and usually at least about 100 spots/cm ², where the density may be as high as 10⁶ or higher, or about 10⁵ spots/cm². In other embodiments, the polymeric sequences are not arranged in the form of distinct spots, but may be positioned on the surface such that there is substantially no space separating one polymer sequence/feature from another. The density of nucleic acids within an individual feature on the array may be as high as 1,000, 10,000, 25,000, 50,000, 100,000, 500,000, 1,000,000, or higher per square micron depending on the intended use of the array.

In some embodiments, the arrays are arrays of nucleic acids, including oligonucleotides, polynucleotides, cDNAs, mRNAs, synthetic mimetics thereof, and the like. Where the arrays are arrays of nucleic acids, the nucleic acids may be covalently attached to the arrays at any point along the nucleic acid chain, but are generally attached at one of their termini, e.g. the 3' or 5' terminus.

Arrays can be used to measure large numbers of analytes or proxies thereof simultaneously. In some embodiments, oligonucleotides are used, at least in part, to create an array. For example, one or more copies of a single species of oligonucleotide (e.g., capture probe) can correspond to or be directly or indirectly attached to a given feature in the array. In some embodiments, a given feature in the array includes two or more species of oligonucleotides (e.g., capture probes). In some embodiments, the two or more species of oligonucleotides (e.g., capture probes) attached directly or indirectly to a given feature on the array include a common (e.g., identical) spatial barcode.

In some embodiments, an array can include a capture probe attached directly or indirectly to the substrate. The capture probe can include a capture domain (e.g., a nucleotide or amino acid sequence) that can specifically bind (e.g., hybridize) to a target analyte (e.g., mRNA, DNA, or protein) or a proxy thereof (e.g., a ligation product obtained from templated ligation of a probe pair) within a sample. In some embodiments, the binding of the capture probe to the target or proxy thereof (e.g., hybridization) can be detected and quantified by detection of a visual signal, e.g., a fluorophore, a heavy metal (e.g., silver ion), or chemiluminescent label, which has been incorporated into the target. In some embodiments, the intensity of the visual signal correlates with the relative abundance of each analyte in the biological sample. Since an array can contain thousands or millions of capture probes (or more), an array can interrogate many analytes or proxies thereof, in parallel. In some embodiments, the binding (e.g., hybridization) of the capture probe to the target can be detected and quantified by creation of a molecule (e.g., cDNA from captured mRNA generated using reverse transcription) that is removed from the array, and processed downstream (e.g., sequenced).

Kits for use in analyte detection assays are provided. In some embodiments, the kit at least includes an array as disclosed herein. The kits may further include one or more additional components necessary for carrying out an analyte detection assay, such as sample preparation reagents, buffers, labels, and the like. As such, the kits may include one or more containers such as tubes, vials or bottles, with each container containing a separate component for the assay, and reagents for carrying out an array assay such as a nucleic acid hybridization assay or the like. The kits may also include a denaturation reagent for denaturing the analyte, buffers such as hybridization buffers, wash mediums, enzyme substrates, reagents for generating a labeled target sample such as a labeled target nucleic acid sample, negative and positive controls and written instructions for using the subject array assay and for carrying out an array based assay. The instructions may be printed on a substrate, such as paper or plastic, etc. As such, the instructions may be present in the kits as a package insert, in the labeling of the container of the kit or components thereof (i.e., associated with the packaging or sub-packaging), *etc.*

The subject arrays find use in a variety of different applications, where such applications are generally analyte detection applications in which the presence of a particular analyte or a proxy thereof, in a given sample is detected at least qualitatively, if not quantitatively. Protocols for carrying out such assays are well known to those of skill in the art and need not be described in great detail here. Generally, the sample suspected of comprising the analyte of interest or proxy thereof (e.g., a tissue section) is contacted with an array produced according to the subject methods under conditions sufficient for the analyte (e.g., mRNA) or proxy thereof (e.g., ligation product comprising a polydA tail) to bind to its respective binding pair member (e.g., poly(dT) capture domain) that is present on the array. Thus, if the analyte or proxy of interest is present in the sample, it binds to the array at a site proximal to its complementary binding member and a complex is formed on the array. The presence of this binding complex on the array is then detected, e.g. through use of a signal production system, e.g. an isotopic or fluorescent label present on the analyte, etc., and/or through sequencing of one or more components of the binding complex or a product thereof. The presence of the analyte in the sample is then deduced from the detection of binding complexes on the substrate, or sequence detection and/or analysis (e.g., by sequencing) of molecules indicative of the formation of the binding complex. In some embodiments, RNA molecules (e.g., mRNA) from a sample are captured by oligonucleotides (e.g., capture probes comprising a barcode and a poly(dT) sequence) on an array prepared by a method disclosed herein, cDNA molecules are generated via reverse transcription of the captured RNA molecules, and the cDNA molecules (e.g., a first strand cDNA) or portions or products (e.g., a second strand cDNA synthesized using a template switching oligonucleotide) thereof can be separated from the array and sequenced. Sequencing data obtained from molecules prepared on the array can be used to deduce the presence/absence or an amount of the RNA molecules in the sample.

Specific analyte detection applications of interest include hybridization assays in which the nucleic acid arrays of the present disclosure are employed. In these assays, a sample of target nucleic acids or a tissue section is first prepared, where preparation may include labeling of the target nucleic acids with a label, e.g., a member of a signal producing system. Following sample preparation, the sample is contacted with the array under hybridization conditions, whereby complexes are formed between target nucleic acids that are complementary to probe sequences attached to the array. The formation and/or presence of hybridized complexes is then detected, e.g., by analyzing molecules that are generated following the formation of the hybridized complexes, such as cDNA or a second strand generated from an RNA captured on the array. Specific hybridization assays of interest which may be practiced using the subject arrays include: gene discovery assays, differential gene expression analysis assays; nucleic acid sequencing assays, single nucleotide polymorphisms (SNPs) assays, copy number variation (CNV) assays, tumor infiltrating lymphocyte assays, and the like.

### A. SPATIAL ANALYSIS

In particular embodiments, provided herein are kits and compositions for spatial array-based analysis of biological samples. Array-based spatial analysis methods involve the transfer of one or more analytes or proxies thereof, from a biological sample (e.g., a tissue section) to an array of features on a substrate, where each feature is associated with a unique spatial location on the array. Subsequent analysis of the transferred analytes or proxies thereof, includes determining the identity of the analytes and the spatial location of each analyte within the biological sample. The spatial location of each analyte within the biological sample is determined based on the feature to which each analyte is bound on the array, and the feature's relative spatial location within the array. In some embodiments, the array of features on a substrate comprise a spatial barcode that corresponds to the feature's relative spatial location within the array. Each spatial barcode of a feature may further comprise a fluorophore, to create a fluorescent hybridization array. A feature may comprise UMIs that are generally unique per nucleic acid molecule in the feature -so the number of unique molecules can be estimated, as opposed to an artifact in experiments or PCR amplification bias that drives amplification of smaller, specific nucleic acid sequences.

In particular embodiments, the kits and compositions for spatial array-based analysis provide for the detection of differences in an analyte level (*e.g.,* gene and/or protein expression) within different cells in a tissue of a mammal or within a single cell from a mammal. For example, the kits and compositions can be used to detect the differences in analyte levels (*e.g.,* gene and/or protein expression) within different cells in histological slide samples (e.g., tissue section), the data from which can be reassembled to generate a three-dimensional map of analyte levels (*e.g.,* gene and/or protein expression) of a tissue sample obtained from a mammal, *e.g.,* with a degree of spatial resolution (*e.g*., single-cell scale resolution).

In some embodiments, an array generated using a method disclosed herein can be used in array-based spatial analysis methods which involve the transfer of one or more analytes from a biological sample to an array of features on a substrate, each of which is associated with a unique spatial location on the array. Subsequent analysis of the transferred analytes includes determining the identity of the analytes and the spatial location of each analyte within the sample. The spatial location of each analyte within the sample is determined based on the feature to which each analyte is bound in the array, and the feature's relative spatial location within the array.

There are at least two general methods to associate a spatial barcode with one or more neighboring cells, such that the spatial barcode identifies the one or more cells, and/or contents of the one or more cells, as associated with a particular spatial location. One general method is to drive target analytes out of a cell and towards the spatially-barcoded array. In some embodiments, the spatially-barcoded array populated with capture probes is contacted with a sample, and sample is permeabilized, allowing the target analyte or proxy thereof, to migrate away from the sample and toward the array. The target analyte (or proxy thereof) interacts with a capture probe on the spatially-barcoded array. Once the target analyte hybridizes/is bound to the capture probe, the sample is optionally removed from the array and the capture probes are analyzed in order to obtain spatially-resolved analyte information. Methods for performing such spatial analysis of tissue sections are known in the art and include but are not limited to those methods disclosed in US Patent 10,030,261, US Patent 11,332,790 and US Patent Pub No. 20220127672 and US Patent Pub No. 20220106632.

Another general method is to cleave the spatially-barcoded capture probes from an array, and drive the spatially-barcoded capture probes towards and/or into or onto the sample. In some embodiments, the spatially-barcoded array populated with capture probes is contacted with a sample. The spatially-barcoded capture probes are cleaved and then interact with cells within the provided sample. See, for example, US Patent 11,352,659. The interaction can be a covalent or non-covalent cell-surface interaction. The interaction can be an intracellular interaction facilitated by a delivery system or a cell penetration peptide. Once the spatially-barcoded capture probe is associated with a particular cell, the sample can be optionally removed for analysis. The sample can be optionally dissociated before analysis. Once the tagged cell is associated with the spatially-barcoded capture probe, the capture probes can be analyzed (e.g., by sequencing) to obtain spatially-resolved information about the tagged cell.

Sample preparation may include placing the sample on a slide, fixing the sample, and/or staining the sample for imaging. The stained sample may be imaged on the array using both brightfield (to image the sample hematoxylin and eosin stain) and/or fluorescence (to image features) modalities. In some embodiments, target analytes are then released from the sample and capture probes forming the spatially-barcoded array hybridize or bind the released target analytes. The sample is then removed from the array and the capture probes cleaved from the array. The sample and array are then optionally imaged a second time in one or both modalities (brightfield and fluorescence) while the analytes are reverse transcribed into cDNA, and an amplicon library is prepared and sequenced. Image(s) can then be spatially-overlaid in order to correlate spatially-identified sample information with sequencing data (e.g., gene expression information). When the sample and array are not imaged a second time, a spot coordinate file may be supplied. The spot coordinate file can replace the second imaging step. Further, amplicon library preparation can be performed with a unique PCR adapter and sequenced.

In some embodiments, a spatially-labelled array on a substrate is used, where capture probes labelled with spatial barcodes are clustered at areas called features. The spatially-labelled capture probes can include a cleavage domain, one or more functional sequences, a spatial barcode, a unique molecular identifier, and a capture domain. The spatially-labelled capture probes can also include a 5' end modification for reversible attachment to the substrate. The spatially-barcoded array is contacted with a sample, and the sample is permeabilized through application of permeabilization reagents. Permeabilization reagents may be administered by placing the array/sample assembly within a bulk solution. Alternatively, permeabilization reagents may be administered to the sample via a diffusion-resistant medium and/or a physical barrier such as a lid, wherein the sample is sandwiched between the diffusion-resistant medium and/or barrier and the array-containing substrate. The analytes are migrated toward the spatially-barcoded capture array using any number of techniques disclosed herein. For example, analyte migration can occur using a diffusion-resistant medium lid and passive migration. As another example, analyte migration can be active migration, using an electrophoretic transfer system, for example. Once the analytes are in close proximity to the spatially-barcoded capture probes, the capture probes can hybridize or otherwise bind a target analyte. The sample can then be optionally removed from the array.

Adapters and assay primers can be used to allow the capture probe or the analyte capture agent to be attached to any suitable assay primers and used in any suitable assays. A capture probe that includes a spatial barcode can be attached to a bead that includes a poly(dT) sequence. A capture probe including a spatial barcode and a poly(T) sequence can be used to assay multiple biological analytes as generally described herein (e.g., the biological analyte includes a poly(A) sequence or is coupled to or otherwise is associated with an analyte capture agent comprising a poly(A) sequence as the analyte capture sequence).

The capture probes can be optionally cleaved from the array, and the captured analytes can be spatially-tagged by performing a reverse transcriptase first strand cDNA reaction. A first strand cDNA reaction can be optionally performed using template switching oligonucleotides. For example, a template switching oligonucleotide can hybridize to a poly(C) tail added to a 3'end of the cDNA by a reverse transcriptase enzyme. The original mRNA template and template switching oligonucleotide can then be denatured from the cDNA and the barcoded capture probe can then hybridize with the cDNA and a complement of the cDNA can be generated. The first strand cDNA can then be purified and collected for downstream amplification steps. The first strand cDNA can be amplified using PCR, wherein the forward and reverse primers flank the spatial barcode and target analyte regions of interest, generating a library associated with a particular spatial barcode. In some embodiments, the cDNA comprises a sequencing by synthesis (SBS) primer sequence. The library amplicons are sequenced and analyzed to decode spatial information.

In some embodiments, the sample is removed from the spatially-barcoded array and the spatially-barcoded capture probes are removed from the array for barcoded analyte amplification and library preparation. Another embodiment includes performing first strand synthesis using template switching oligonucleotides on the spatially-barcoded array without cleaving the capture probes. Once the capture probes capture the target analyte(s), first strand cDNA created by template switching and reverse transcriptase is then denatured and the second strand is then extended. The second strand cDNA is then denatured from the first strand cDNA, and transferred to a separate vessel (e.g., tube). cDNA quantification and amplification can be performed using standard techniques discussed herein. The cDNA can then be subjected to library preparation and indexing, including fragmentation, end-repair, A-tailing, indexing PCR steps, and then sequenced.

### VI. TERMINOLOGY

Unless defined otherwise, all terms of art, notations and other technical and scientific terms or terminology used herein are intended to have the same meaning as is commonly understood by one of ordinary skill in the art to which the claimed subject matter pertains. In some cases, terms with commonly understood meanings are defined herein for clarity and/or for ready reference, and the inclusion of such definitions herein should not necessarily be construed to represent a substantial difference over what is generally understood in the art.

Throughout this disclosure, various aspects of the claimed subject matter are presented in a range format. It should be understood that the description in range format is merely for convenience and brevity and should not be construed as an inflexible limitation on the scope of the claimed subject matter. Accordingly, the description of a range should be considered to have specifically disclosed all the possible sub-ranges as well as individual numerical values within that range. For example, where a range of values is provided, it is understood that each intervening value, between the upper and lower limit of that range and any other stated or intervening value in that stated range is encompassed within the claimed subject matter. The upper and lower limits of these smaller ranges may independently be included in the smaller ranges, and are also encompassed within the claimed subject matter, subject to any specifically excluded limit in the stated range. Where the stated range includes one or both of the limits, ranges excluding either or both of those included limits are also included in the claimed subject matter. This applies regardless of the breadth of the range.

Use of ordinal terms such as "first", "second", "third", etc., in the claims to modify a claim element does not by itself connote any priority, precedence, or order of one claim element over another or the temporal order in which acts of a method are performed, but are used merely as labels to distinguish one claim element having a certain name from another element having a same name (but for use of the ordinal term) to distinguish the claim elements. Similarly, use of a), b), etc., or i), ii), etc. does not by itself connote any priority, precedence, or order of steps in the claims. Similarly, the use of these terms in the specification does not by itself connote any required priority, precedence, or order.

As used in this specification and the appended claims, the singular forms "a," "an" and "the" include plural referents unless the context clearly dictates otherwise. Thus, for example, reference to "a molecule" includes a plurality of such molecules, and the like.

A sample such as a biological sample can include any number of macromolecules, for example, cellular macromolecules and organelles (e.g., mitochondria and nuclei). The biological sample can be a nucleic acid sample and/or protein sample. The biological sample can be a carbohydrate sample or a lipid sample. The biological sample can be obtained as a tissue sample, such as a tissue section, biopsy, a core biopsy, needle aspirate, or fine needle aspirate. The sample can be a fluid sample, such as a blood sample, urine sample, or saliva sample. The sample can be a skin sample, a colon sample, a cheek swab, a histology sample, a histopathology sample, a plasma or serum sample, a tumor sample, living cells, cultured cells, a clinical sample such as, for example, whole blood or blood-derived products, blood cells, or cultured tissues or cells, including cell suspensions. In some embodiments, the biological sample may comprise cells which are deposited on a surface.

The term "barcode," comprises a label, or identifier, that conveys or is capable of conveying information (e.g., information about an analyte in a sample, a bead, and/or a capture probe). A barcode can be part of an analyte, or independent of an analyte. A barcode can be attached to an analyte. A particular barcode can be unique relative to other barcodes. Barcodes can have a variety of different formats. For example, barcodes can include polynucleotide barcodes, random nucleic acid and/or amino acid sequences, and synthetic nucleic acid and/or amino acid sequences. A barcode can be attached to an analyte or to another moiety or structure in a reversible or irreversible manner. A barcode can be added to, for example, a fragment of a deoxyribonucleic acid (DNA) or ribonucleic acid (RNA) sample before or during sequencing of the sample. Barcodes can allow for identification and/or quantification of individual sequencing-reads (e.g., a barcode can be or can include a unique molecular identifier or "UMI").

Barcodes can spatially-resolve molecular components found in biological samples, for example, at single-cell scale resolution (e.g., a barcode can be or can include a "spatial barcode"). In some embodiments, a barcode includes both a UMI and a spatial barcode. In some embodiments, a barcode includes two or more sub-barcodes that together function as a single barcode. For example, a polynucleotide barcode can include two or more polynucleotide sequences (*e*.*g*., sub-barcodes) that are separated by one or more non-barcode sequences.

As used herein, the term "substrate" generally refers to a substance, structure, surface, material, means, or composition, which comprises a nonbiological, synthetic, nonliving, planar, spherical or flat surface. The substrate may include, for example and without limitation, semiconductors, synthetic metals, synthetic semiconductors, insulators and dopants; metals, alloys, elements, compounds and minerals; synthetic, cleaved, etched, lithographed, printed, machined and microfabricated slides, wafers, devices, structures and surfaces; industrial polymers, plastics, membranes; silicon, silicates, glass, metals and ceramics; wood, paper, cardboard, cotton, wool, cloth, woven and nonwoven fibers, materials and fabrics; nanostructures and microstructures. The substrate may comprise an immobilization matrix such as but not limited to, insolubilized substance, solid phase, surface, layer, coating, woven or nonwoven fiber, matrix, crystal, membrane, insoluble polymer, plastic, glass, biological or biocompatible or bioerodible or biodegradable polymer or matrix, microparticle or nanoparticle. Other examples may include, for example and without limitation, monolayers, bilayers, commercial membranes, resins, matrices, fibers, separation media, chromatography supports, polymers, plastics, glass, mica, gold, beads, microspheres, nanospheres, silicon, gallium arsenide, organic and inorganic metals, semiconductors, insulators, microstructures and nanostructures. Microstructures and nanostructures may include, without limitation, microminiaturized, nanometer-scale and supramolecular probes, tips, bars, pegs, plugs, rods, sleeves, wires, filaments, and tubes.

As used herein, the term "nucleic acid" generally refers to a polymer comprising one or more nucleic acid subunits or nucleotides. A nucleic acid may include one or more subunits selected from adenosine (A), cytosine (C), guanine (G), thymine (T) and uracil (U), or variants thereof. A nucleotide can include A, C, G, T or U, or variants thereof. A nucleotide can include any subunit that can be incorporated into a growing nucleic acid strand. Such subunit can be an A, C, G, T, or U, or any other subunit that is specific to one or more complementary A, C, G, T or U, or complementary to a purine (i.e., A or G, or variant thereof) or a pyrimidine (i.e., C, T or U, or variant thereof). A subunit can enable individual nucleic acid bases or groups of bases (e.g., AA, TA, AT, GC, CG, CT, TC, GT, TG, AC, CA, or uracil-counterparts thereof) to be resolved. In some examples, a nucleic acid is deoxyribonucleic acid (DNA) or ribonucleic acid (RNA), or derivatives thereof. A nucleic acid may be single-stranded or double-stranded.

The term "nucleic acid sequence" or "nucleotide sequence" as used herein generally refers to nucleic acid molecules with a given sequence of nucleotides, of which it may be desired to know the presence or amount. The nucleotide sequence can comprise ribonucleic acid (RNA) or DNA, or a sequence derived from RNA or DNA. Examples of nucleotide sequences are sequences corresponding to natural or synthetic RNA or DNA including genomic DNA and messenger RNA. The length of the sequence can be any length that can be amplified into nucleic acid amplification products, or amplicons, for example, up to about 20, 50, 100, 200, 300, 400, 500, 600, 700, 800, 1000, 1200, 1500, 2000, 5000, 10000 or more than 10000 nucleotides in length, or at least about 20, 50, 100, 200, 300, 400, 500, 600, 700, 800, 1000, 1200, 1500, 2000, 5000, 10000 nucleotides in length.

The terms "oligonucleotide" and "polynucleotide" are used interchangeably to refer to a single-stranded multimer of nucleotides from about 2 to about 500 nucleotides in length. Oligonucleotides can be synthetic, made enzymatically (e.g., via polymerization), or using a "split-pool" method. Oligonucleotides can include ribonucleotide monomers (i.e., can be oligoribonucleotides) and/or deoxyribonucleotide monomers (i.e., oligodeoxyribonucleotides). In some examples, oligonucleotides can include a combination of both deoxyribonucleotide monomers and ribonucleotide monomers in the oligonucleotide (e.g., random or ordered combination of deoxyribonucleotide monomers and ribonucleotide monomers). An oligonucleotide can be 4 to 10, 10 to 20, 21 to 30, 31 to 40, 41 to 50, 51 to 60, 61 to 70, 71 to 80, 80 to 100, 100 to 150, 150 to 200, 200 to 250, 250 to 300, 300 to 350, 350 to 400, or 400-500 nucleotides in length, for example. Oligonucleotides can include one or more functional moieties that are attached (e.g., covalently or non-covalently) to the multimer structure. For example, an oligonucleotide can include one or more detectable labels (e.g., a radioisotope or fluorophore).

As used herein, the term "adjacent" or "adjacent to," includes "next to," "adjoining," and "abutting." In one example, a first location is adjacent to a second location when the first location is in direct contact and shares a common border with the second location and there is no space between the two locations. In some cases, the adjacent is not diagonally adjacent.

An "adaptor," an "adapter," and a "tag" are terms that are used interchangeably in this disclosure, and refer to species that can be coupled to a polynucleotide sequence (in a process referred to as "tagging") using any one of many different techniques including (but not limited to) ligation, hybridization, and tagmentation. Adaptors can also be nucleic acid sequences that add a function, e.g., spacer sequences, primer sequences/sites, barcode sequences, unique molecular identifier sequences.

The terms "hybridizing," "hybridize," "annealing," and "anneal" are used interchangeably in this disclosure, and refer to the pairing of substantially complementary or complementary nucleic acid sequences within two different molecules. Pairing can be achieved by any process in which a nucleic acid sequence joins with a substantially or fully complementary sequence through base pairing to form a hybridization complex. For purposes of hybridization, two nucleic acid sequences are "substantially complementary" if at least 60% (e.g., at least 70%, at least 80%, or at least 90%) of their individual bases are complementary to one another.

A "proximity ligation" is a method of ligating two (or more) nucleic acid sequences that are in proximity with each other through enzymatic means (e.g., a ligase). In some embodiments, proximity ligation can include a "gap-filling" step that involves incorporation of one or more nucleic acids by a polymerase, based on the nucleic acid sequence of a template nucleic acid molecule, spanning a distance between the two nucleic acid molecules of interest (see, e.g., U.S. Patent No. 7,264,929).

A wide variety of different methods can be used for proximity ligating nucleic acid molecules, including (but not limited to) "sticky-end" and "blunt-end" ligations. Additionally, single-stranded ligation can be used to perform proximity ligation on a single-stranded nucleic acid molecule. Sticky-end proximity ligations involve the hybridization of complementary single-stranded sequences between the two nucleic acid molecules to be joined, prior to the ligation event itself. Blunt-end proximity ligations generally do not include hybridization of complementary regions from each nucleic acid molecule because both nucleic acid molecules lack a single-stranded overhang at the site of ligation

As used herein, the term "splint" is an oligonucleotide that, when hybridized to other polynucleotides, acts as a "splint" to position the polynucleotides next to one another so that they can be ligated together. In some embodiments, the splint is DNA or RNA. The splint can include a nucleotide sequence that is partially complimentary to nucleotide sequences from two or more different oligonucleotides. In some embodiments, the splint assists in ligating a "donor" oligonucleotide and an "acceptor" oligonucleotide. In general, an RNA ligase, a DNA ligase, or another other variety of ligase is used to ligate two nucleotide sequences together.

In some embodiments, the splint is between 6 and 50 nucleotides in length, e.g., between 6 and 45, 6 and 40, 6 and 35, 6 and 30, 6 and 25, or 6 and 20 nucleotides in length. In some embodiments, the splint is between 10 and 50 nucleotides in length, e.g., between 10 and 45, 10 and 40, 10 and 35, 10 and 30, 10 and 25, or 10 and 20 nucleotides in length. In some embodiments, the splint is between 15 and 50, 15 and 45, 15 and 40, 15 and 35, 15 and 30, or 15 and 25 nucleotides in length.

A "feature" is an entity that acts as a support or repository for various molecular entities used in sample analysis. In some embodiments, some or all of the features in an array are functionalized for analyte capture. In some embodiments, functionalized features include one or more capture probe(s). Examples of features include, but are not limited to, a bead, a spot of any two- or three-dimensional geometry (e.g., an ink jet spot, a masked spot, a square on a grid), a well, and a hydrogel pad. In some embodiments, features are directly or indirectly attached or fixed to a substrate. In some embodiments, the features are not directly or indirectly attached or fixed to a substrate, but instead, for example, are disposed within an enclosed or partially enclosed three dimensional space (e.g., wells or divots).

The term "sequencing," as used herein, generally refers to methods and technologies for determining the sequence of nucleotide bases in one or more polynucleotides. The polynucleotides can be, for example, nucleic acid molecules such as deoxyribonucleic acid (DNA) or ribonucleic acid (RNA), including variants or derivatives thereof (e.g., single stranded DNA). Sequencing can be performed by various systems currently available, such as, without limitation, a sequencing system by Illumina^{®}, Pacific Biosciences (PacBio^{®}), Oxford Nanopore^{®}, or Life Technologies (Ion Torrent^{®}). Alternatively or in addition, sequencing may be performed using nucleic acid amplification, polymerase chain reaction (PCR) (e.g., digital PCR, quantitative PCR, or real time PCR), or isothermal amplification. Such systems may provide a plurality of raw genetic data corresponding to the genetic information of a subject (e.g., human), as generated by the systems from a sample provided by the subject. In some examples, such systems provide sequencing reads (also "reads" herein). A read may include a string of nucleic acid bases corresponding to a sequence of a nucleic acid molecule that has been sequenced. In some situations, systems and methods provided herein may be used with proteomic information.

The term "template" as used herein generally refers to individual polynucleotide molecules from which another nucleic acid, including a complementary nucleic acid strand, can be synthesized by a nucleic acid polymerase. In addition, the template can be one or both strands of the polynucleotides that are capable of acting as templates for template-dependent nucleic acid polymerization catalyzed by the nucleic acid polymerase. Use of this term should not be taken as limiting the scope of the present disclosure to polynucleotides which are actually used as templates in a subsequent enzyme-catalyzed polymerization reaction. The template can be an RNA or DNA. The template can be cDNA corresponding to an RNA sequence. The template can be DNA.

As used herein, "amplification" of a template nucleic acid generally refers to a process of creating (e.g., in vitro) nucleic acid strands that are identical or complementary to at least a portion of a template nucleic acid sequence, or a universal or tag sequence that serves as a surrogate for the template nucleic acid sequence, all of which are only made if the template nucleic acid is present in a sample. Typically, nucleic acid amplification uses one or more nucleic acid polymerase and/or transcriptase enzymes to produce multiple copies of a template nucleic acid or fragments thereof, or of a sequence complementary to the template nucleic acid or fragments thereof. In vitro nucleic acid amplification techniques are may include transcription-associated amplification methods, such as Transcription-Mediated Amplification (TMA) or Nucleic Acid Sequence-Based Amplification (NASBA), and other methods such as Polymerase Chain Reaction (PCR), Reverse Transcriptase-PCR (RT-PCR), Replicase Mediated Amplification, and Ligase Chain Reaction (LCR).

In addition to those above, a wide variety of other features can be used to form the arrays described herein. For example, in some embodiments, features that are formed from polymers and/or biopolymers that are jet printed, screen printed, or electrostatically deposited on a substrate can be used to form arrays.

The present disclosure is not intended to be limited in scope to the particular disclosed embodiments, which are provided, for example, to illustrate various aspects of the present disclosure. Various modifications to the compositions and methods described will become apparent from the description and teachings herein. Such variations may be practiced without departing from the true scope and spirit of the disclosure and are intended to fall within the scope of the present disclosure.

### EXAMPLES

The following example is included for illustrative purposes only and is not intented to limit the scope of the present disclosure.

### Example 1: Generation of an oligonucleotide array using 3' capping or Exonuclease I digestion.

This Example demonstrates a successful assembly of barcoded oligonucleotides following photoresist, on a lawn of immobilized oligonucleotides (e.g., a lawn of partial read 1 oligonucleotides). In particular, this Example describes the generation of an array with higher resolution and/or higher barcode accuracy by blocking or digesting the boundary region between the features (e.g., spots).

A lawn of partial Read 1 (pR1) oligonucleotides were immobilized on a glass slide. A photoresist was applied on the surface of the slide and a photo-hybridization/ligation reaction was initated. Based on the photopatterning used, only certain subpopulations of immobilized oligonucleotides should have been available for subsequent photo-hybridization/ligation reactions. However, pR1 oligonucleotides beyond the dimensions of the patterning (e.g., features) were capable of non-specific hybridization and ligation reactions. An improved method to control the feature size and resolution and eliminate non-specific erroneous barcoding of oligonucleotides in the boundary region was needed.

**FIG. 4** illustrates an exemplary method for generating an array, where the feature size is highly controlled. Lawns of pR1 oligonucleotides were immobilized on six glass slides. The surfaces of the glass substrates were coated with a photoresist. A photoresist pattern was generated on slides 1 and 2 (as shown in Table 1) by irradiating the substrate through a patterned photomask that covered the boundary regions between the features, while exposing the features (e.g., spots) to degradation. Slides 3-6 (as shown in Table 1) comprised a photoresist pattern whereby the unmasked boundary region was exposed to degradation while the masked spots were covered by a photomask. The photoresist in the boundary region was degraded to render the boundary region available for blocking or digestion of oligonucleotides in the boundary region. As shown in **FIG. 4**, the oligonucleotides in the boundary regions were then blocked via 3' capping with dideoxy nucleotides (slides 3 and 4)) or digested using Exonuclease I (slides 5 and 6). Next, the photoresist from the slides 3-6 was stripped using acetone. A four-part photo-hybridization/ligation reaction comprising four cycles was performed on all slides. Each cycle comprised attaching a barcode to the immobilized oligonucleotides in the spots. Each attaching step hybridized a splint to the immobilized oligonucleotides in the spots and oligonucleotides comprising barcode sequences were added, followed by ligating the oligonucleotides, to generate an extended oligonucleotide (as shown in **FIG. 4**).

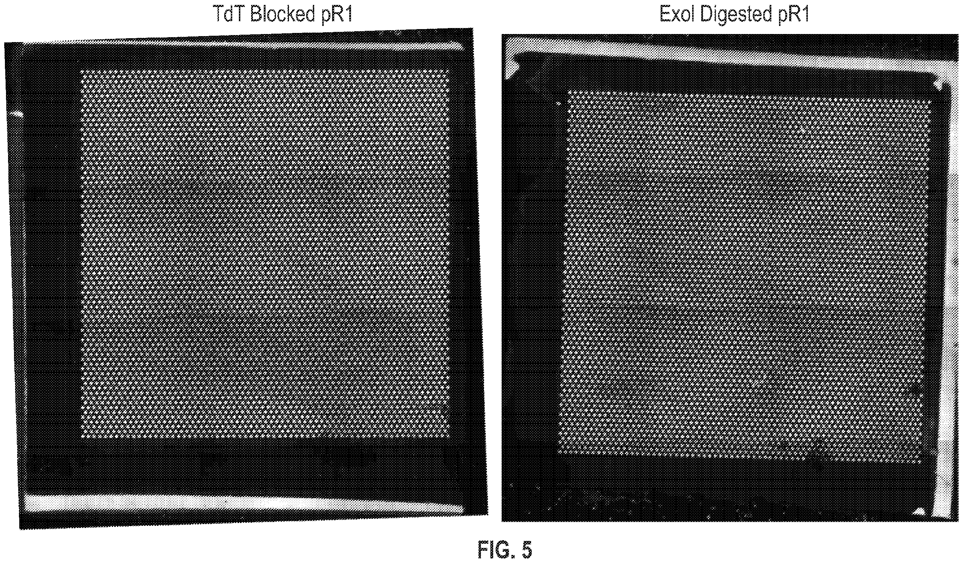

Once the four-part hybridization/ligation method was performed on slides 3-6, the photoresist from slides 1 and 2 was stripped using acetone. All the slides were then soaked in a developer solution to solubilize the degraded photoresist. The next day, fluorescent probes were hybridized to the four-part ligation products on slides 1-6 and imaged. **FIG. 5** shows two fluorescent arrays generated by the method described herein via 3' capping and Exonuclease I digestion. As demonstrated in **Table 1**, the spatially barcoded features generated from the method described herein were about 55 microns in diameter. Additionally, by performing blocking or Exonuclease I mediated digestion of the pR1 oligonucleotides in the boundary region, the background noise (signal-to-noise ratio) did not increase beyond the control (non-capped or digested slides 1 and 2).

| Table 1. Summary of results from the exemplary method of FIG. 4 | | | | |
|---|---|---|---|---|
| Slide number | Sample | Repeat | Diameter (microns) | Signal-to-Noise ratio |
| 1 | Free pR1 oligonucleotides | 1 | 55 ± 2 | 13 ± 1 |
| 2 | Free pR1 oligonucleotides | 2 | 55 ± 2 | 12 ± 1 |
| 3 | Terminal deoxynucleotidyl transferase (TdT) | 1 | 53 ± 1 | 12 ± 1 |
| 4 | TdT | 2 | 53 ± 2 | 11 ± 1 |
| 5 | Exonuclease I | 1 | 53 ± 2 | 12 ± 1 |
| 6 | Exonuclease I | 2 | 53 ± 2 | 12 ± 1 |

### Example 2: Exemplary workflow for sample analysis using an array.

This Example describes an exemplary workflow for analyzing a biological sample (e.g., a tissue section) using an array as disclosed herein.

An array can be generated according to any of the embodiments described herein (e.g., as described in Example 1).

A tissue section is deposited on the array and processed. The tissue section is stained, and then permeabilized to capture mRNA molecules from the tissue section on the array via the polyT sequences of the capture domains in the immobilized oligonucleotides. First strand cDNAs are created on the array via reverse transcription of the captured mRNAs for 10 mins at room temperature. Second strand synthesis is performed via template switching to generate second strand cDNAs. The second strand cDNAs are then amplified. The next day, the cDNA library is fragmented, ligated to sequencing adaptors, and sequenced.

## Claims

1. A method for providing an array, comprising:
a) irradiating a substrate comprising a plurality of masked spot regions and an unmasked boundary region between the plurality of masked spot regions, whereby a photoresist in the boundary region is degraded to render the boundary region available for blocking or ablation of oligonucleotide molecules in the boundary region, whereas the spot regions are protected by a photoresist in the spot regions from oligonucleotide blocking or ablation;
b) blocking or ablating oligonucleotide molecules in the boundary region;
c) irradiating the substrate to render an oligonucleotide molecule immobilized in a first one or more of the spot regions on the substrate available for oligonucleotide attachment, whereas an oligonucleotide molecule immobilized in one or more other spot regions on the substrate are not available for oligonucleotide attachment; and
d) attaching a first oligonucleotide of at least four nucleotides in length to the oligonucleotide molecule immobilized in the first one or more of the spot regions to provide an extended oligonucleotide molecule in the first one or more of the spot regions.

2. The method of claim 1, wherein the irradiating in step a) and blocking in step b) are performed before the irradiating in step c) and attaching in step d).

3. The method of claim 1 or 2, comprising blocking oligonucleotide molecules in the boundary region by adding a 3' dideoxy, a non-ligating 3' phosphoramidate, or a triphenylmethyl (trityl) group to the oligonucleotide molecules in the boundary region, optionally wherein the addition is catalyzed by a terminal transferase, optionally wherein the terminal transferase is a terminal deoxynucleotidyl transferase (TdT).

4. The method of claim 1 or 2, comprising ablating oligonucleotide molecules in the boundary region,
optionally wherein ablating oligonucleotide molecules in the boundary region comprises digesting the oligonucleotide molecules with an exonuclease, optionally wherein the exonuclease is Exonuclease I, or
optionally wherein ablating oligonucleotide molecules in the boundary region comprises performing plasma etching in the boundary region, optionally wherein the plasma etching comprises exposing the boundary region to oxygen-based plasma, or
optionally wherein ablating oligonucleotide molecules completely removes exposed oligonucleotide molecules in the boundary region.

5. The method of any one of claims 1-4, wherein prior to the irradiation in step c), the oligonucleotide molecules in the first one or more of the spot regions are protected from hybridization and/or ligation,
optionally wherein the oligonucleotide molecules in the spot region(s) are protected from hybridization and/or ligation by a photoresist covering the oligonucleotide molecules,
optionally wherein the photoresist in irradiated spot regions is removed and the photoresist in masked or non-irradiated spot regions is not removed.

6. The method of any one of claims 1-5, wherein during and after the irradiation in step c), the oligonucleotide molecules in the one or more other spot regions are protected from hybridization and/or ligation,
optionally wherein the oligonucleotide molecules in the spot region(s) are protected from hybridization and/or ligation by a photoresist covering the oligonucleotide molecules,
optionally wherein the photoresist in irradiated spot regions is removed and the photoresist in masked or non-irradiated spot regions is not removed.

7. The method of any one of claims 1-6, wherein
i) the oligonucleotide molecules in the spot region(s) are protected from hybridization and/or ligation by a protective group of each oligonucleotide molecule,
optionally wherein the protective group is a photo-cleavable protective group, optionally wherein the photo-cleavable protective groups in irradiated regions in step c) are cleaved and the photo-cleavable protective groups in masked or non-irradiated regions in step c) are not cleaved, or
ii) the oligonucleotide molecules in the spot region(s) are protected from hybridization and/or ligation by a polymer binding to the oligonucleotide molecules,
optionally wherein the polymer is a photo-cleavable polymer, optionally wherein the polymer binds to the oligonucleotide molecules in a non-sequence specific manner,
optionally wherein the photo-cleavable polymers in the irradiated first one or more spot of the spot regions are cleaved and the photo-cleavable polymers in masked or non-irradiated one or more other spot regions are not cleaved.

8. The method of any one of claims 1-7, comprising irradiating the substrate in multiple cycles, each cycle for irradiating one or more spot regions that are different from the one or more spot region(s) irradiated in another cycle and attaching an oligonucleotide of at least four nucleotides in length to the oligonucleotide molecule immobilized in irradiated spot region(s) for the cycle.

9. The method of any one of claims 1-8, wherein the irradiating in step c) is performed using a photomask, wherein the first one or more of the spot regions are unmasked and the one or more other spot regions are masked.

10. The method of claim 9, comprising translating the photomask used in the irradiating step in c) from a first position to a second position relative to the substrate, each position for a cycle of irradiating the substrate and attaching an oligonucleotide.

11. The method of any one of claims 1-10, wherein oligonucleotide molecules in the first one or more of the spot regions, the one or more other spot regions, and the boundary region are identical in sequence prior to the irradiating in step a).

12. The method of any one of claims 1-11, wherein the plurality of spot regions are, on average, no more than 55 micrometers in diameter,
optionally wherein the plurality of spot regions are, on average, no more than 5 micrometers in diameter.

13. The method of any one of claims 1-12, wherein the boundary region comprises the region on the substrate surrounding and between the plurality of spot regions.

14. The method of any one of claims 1-13, wherein oligonucleotide molecules on the substrate prior to the irradiating step are between about 4 and about 50 nucleotides in length.

15. The method of any one of claims 1-14, wherein the oligonucleotide molecules are applied to the substrate as a lawn prior to the irradiating in step a).

## Patentansprüche

1. Verfahren zum Bereitstellen eines Arrays, umfassend:
a) Bestrahlen eines Substrats, das eine Vielzahl von maskierten Punktregionen und eine unmaskierte Grenzregion zwischen der Vielzahl von maskierten Punktregionen umfasst, wobei ein Fotolack in der Grenzregion abgebaut wird, um die Grenzregion für das Blockieren oder die Ablation von Oligonukleotidmolekülen in der Grenzregion verfügbar zu machen, während die Punktregionen durch einen Fotolack in den Punktregionen gegen Oligonukleotidblockierung oder -ablation geschützt sind;
b) Blockieren oder Ablatieren von Oligonukleotidmolekülen in der Grenzregion;
c) Bestrahlen des Substrats, um ein Oligonukleotidmolekül, das in einer ersten oder mehreren der Punktregionen auf dem Substrat immobilisiert ist, für das Anlagern von Oligonukleotiden verfügbar zu machen, während ein Oligonukleotidmolekül, das in einer oder mehreren anderen Punktregionen auf dem Substrat immobilisiert ist, für das Anlagern von Oligonukleotiden nicht verfügbar ist; und
d) Anlagern eines ersten Oligonukleotids mit einer Länge von zumindest vier Nukleotiden an das in der ersten einen oder den mehreren der Punktregionen immobilisierte Oligonukleotidmolekül, um ein verlängertes Oligonukleotidmolekül in der ersten einen oder den mehreren der Punktregionen bereitzustellen.

2. Verfahren nach Anspruch 1, wobei das Bestrahlen in Schritt a) und das Blockieren in Schritt b) vor dem Bestrahlen in Schritt c) und dem Anlagern in Schritt d) durchgeführt werden.

3. Verfahren nach Anspruch 1 oder 2, umfassend das Blockieren von Oligonukleotidmolekülen in der Grenzregion durch Zugabe einer 3'-Dideoxy-, einer nicht ligierenden 3'-Phosphoramidat- oder einer Triphenylmethyl(Trityl)-Gruppe zu den Oligonukleotidmolekülen in der Grenzregion, optional, wobei die Zugabe durch eine terminale Transferase katalysiert wird, optional, wobei die terminale Transferase eine terminale Desoxynukleotidyltransferase (TdT) ist.

4. Verfahren nach Anspruch 1 oder 2, umfassend das Ablatieren von Oligonukleotidmolekülen in der Grenzregion,
optional, wobei das Ablatieren von Oligonukleotidmolekülen in der Grenzregion das Verdauen der Oligonukleotidmoleküle mit einer Exonuklease umfasst, wobei die Exonuklease optional Exonuklease I ist, oder
optional, wobei das Ablatieren von Oligonukleotidmolekülen in der Grenzregion das Durchführen einer Plasmaätzung in der Grenzregion umfasst, optional, wobei das Plasmaätzen das Aussetzen der Grenzregion gegenüber einem Plasma auf Sauerstoffbasis umfasst, oder
optional, wobei das Ablatieren von Oligonukleotidmolekülen die exponierten Oligonukleotidmoleküle in der Grenzregion vollständig entfernt.

5. Verfahren nach einem der Ansprüche 1 bis 4, wobei vor dem Bestrahlen in Schritt c) die Oligonukleotidmoleküle in der ersten einen oder den mehreren der Punktregionen gegen Hybridisierung und/oder Ligation geschützt werden,
optional, wobei die Oligonukleotidmoleküle in der Punktregion/den Punktregionen durch einen Fotolack, der die Oligonukleotidmoleküle bedeckt, gegen Hybridisierung und/oder Ligation geschützt sind,
optional, wobei der Fotolack in bestrahlten Punktregionen entfernt wird und der Fotolack in maskierten oder nicht bestrahlten Punktregionen nicht entfernt wird.

6. Verfahren nach einem der Ansprüche 1 bis 5, wobei während und nach der Bestrahlung in Schritt c) die Oligonukleotidmoleküle in den einen oder mehreren anderen Punktregionen gegen Hybridisierung und/oder Ligation geschützt sind,
optional, wobei die Oligonukleotidmoleküle in der Punktregion/den Punktregionen durch einen Fotolack, der die Oligonukleotidmoleküle bedeckt, gegen Hybridisierung und/oder Ligation geschützt sind,
optional, wobei der Fotolack in bestrahlten Punktregionen entfernt wird und der Fotolack in maskierten oder nicht bestrahlten Punktregionen nicht entfernt wird.

7. Verfahren nach einem der Ansprüche 1 bis 6, wobei
i) die Oligonukleotidmoleküle in der Punktregion/den Punktregionen durch eine Schutzgruppe jedes Oligonukleotidmoleküls gegen Hybridisierung und/oder Ligation geschützt sind,
optional, wobei die Schutzgruppe eine fotospaltbare Schutzgruppe ist, optional, wobei die fotospaltbaren Schutzgruppen in bestrahlten Regionen in Schritt c) gespalten werden und die fotospaltbaren Schutzgruppen in maskierten oder nicht bestrahlten Regionen in Schritt c) nicht gespalten werden, oder
ii) die Oligonukleotidmoleküle in der Punktregion/den Punktregionen durch ein an die Oligonukleotidmoleküle bindendes Polymer gegen Hybridisierung und/oder Ligation geschützt sind,
optional, wobei das Polymer ein fotospaltbares Polymer ist, optional, wobei das Polymer an die Oligonukleotidmoleküle in einer nicht sequenzspezifischen Weise bindet,
optional, wobei die fotospaltbaren Polymere in dem bestrahlten ersten einen Punkt oder den mehreren bestrahlten Punkten der Punktregionen gespalten werden und die fotospaltbaren Polymere in einer oder mehreren anderen maskierten oder nicht bestrahlten Punktregionen nicht gespalten werden.

8. Verfahren nach einem der Ansprüche 1 bis 7, umfassend das Bestrahlen des Substrats in mehreren Zyklen, wobei jeder Zyklus zum Bestrahlen einer oder mehrerer Punktregionen dient, die sich von der einen oder den mehreren in einem anderen Zyklus bestrahlten Punktregion(en) unterscheiden, und das Anlagern eines Oligonukleotids mit einer Länge von zumindest vier Nukleotiden an das Oligonukleotidmolekül, das in der bestrahlten Punktregion/den bestrahlten Punktregionen für den Zyklus immobilisiert ist.

9. Verfahren nach einem der Ansprüche 1 bis 8, wobei das Bestrahlen in Schritt c) unter Verwendung einer Fotomaske durchgeführt wird, wobei die erste eine oder mehrere Punktregionen unmaskiert sind und die eine oder mehreren anderen Punktregionen maskiert sind.

10. Verfahren nach Anspruch 9, umfassend das Überführen der in dem Bestrahlungsschritt in c) verwendeten Fotomaske von einer ersten Position in eine zweite Position relativ zu dem Substrat, wobei jede Position für einen Zyklus der Bestrahlung des Substrats und des Anlagerns eines Oligonukleotids vorgesehen ist.

11. Verfahren nach einem der Ansprüche 1 bis 10, wobei die Oligonukleotidmoleküle in der ersten einen oder mehreren der Punktregionen, der einen oder mehreren anderen Punktregionen und der Grenzregion vor dem Bestrahlen in Schritt a) in ihrer Sequenz identisch sind.

12. Verfahren nach einem der Ansprüche 1 bis 11, wobei die Vielzahl der Punktregionen im Durchschnitt einen Durchmesser von nicht mehr als 55 Mikrometer aufweist,
optional, wobei die Vielzahl der Punktregionen im Durchschnitt einen Durchmesser von nicht mehr als 5 Mikrometer aufweist.

13. Verfahren nach einem der Ansprüche 1 bis 12, wobei die Grenzregion die Region auf dem Substrat umfasst, die die Vielzahl von Punktregionen umgibt und zwischen ihnen liegt.

14. Verfahren nach einem der Ansprüche 1 bis 13, wobei die Oligonukleotidmoleküle auf dem Substrat vor dem Bestrahlungsschritt eine Länge zwischen etwa 4 und etwa 50 Nukleotiden aufweisen.

15. Verfahren nach einem der Ansprüche 1 bis 14, wobei die Oligonukleotidmoleküle vor dem Bestrahlen in Schritt a) als Rasen auf das Substrat aufgebracht werden.

## Revendications

1. Procédé pour fournir un réseau, comprenant :
a) l'irradiation d'un substrat comprenant une pluralité de régions de tache masquées et une région limite non masquée entre la pluralité de régions de tache masquées, moyennant quoi une photorésine dans la région limite est dégradée pour rendre la région limite disponible pour le blocage ou l'ablation de molécules d'oligonucléotides dans la région limite, tandis que les régions de tache sont protégées par une photorésine dans les régions de tache contre le blocage ou l'ablation d'oligonucléotides ;
b) le blocage ou l'ablation de molécules d'oligonucléotides dans la région limite ;
c) l'irradiation du substrat pour rendre une molécule d'oligonucléotide immobilisée dans une première ou plusieurs des régions de tache sur le substrat disponible pour la fixation d'oligonucléotides, tandis qu'une molécule d'oligonucléotide immobilisée dans une ou plusieurs autres régions de tache sur le substrat ne sont pas disponibles pour la fixation d'oligonucléotides ; et
d) la fixation d'un premier oligonucléotide de l'au moins quatre nucléotides de longueur à la molécule d'oligonucléotide immobilisée dans la première ou plusieurs des régions de tache pour fournir une molécule d'oligonucléotide étendue dans la première ou plusieurs des régions de point.

2. Procédé selon la revendication 1, dans lequel l'irradiation à l'étape a) et le blocage à l'étape b) sont réalisés avant l'irradiation à l'étape c) et la fixation à l'étape d).

3. Procédé selon la revendication 1 ou 2, comprenant le blocage de molécules d'oligonucléotides dans la région limite par addition d'un 3'-didésoxy, d'un 3'-phosphoramidate non ligaturant ou d'un groupe triphénylméthyle (trityle) aux molécules d'oligonucléotides dans la région limite, facultativement dans lequel l'addition est catalysée par une transférase terminale, facultativement dans lequel la transférase terminale est un une désoxynucléotide transférase terminale (dTd).

4. Procédé selon la revendication 1 ou 2, comprenant l'ablation de molécules d'oligonucléotides dans la région limite,
facultativement dans lequel l'ablation de molécules d'oligonucléotides dans la région limite comprend la digestion des molécules d'oligonucléotides avec une exonucléase, facultativement dans lequel l'exonucléase est l'Exonucléase I, ou
facultativement dans lequel l'ablation de molécules d'oligonucléotides dans la région limite comprend la réalisation d'une gravure au plasma dans la région limite, facultativement dans lequel la gravure au plasma comprend l'exposition de la région limite à un plasma à base d'oxygène, ou
facultativement dans lequel l'ablation de molécules d'oligonucléotides élimine complètement les molécules d'oligonucléotides exposées dans la région limite.

5. Procédé selon l'une quelconque des revendications 1 à 4, dans lequel, avant l'irradiation à l'étape c), les molécules d'oligonucléotides dans la première ou plusieurs des régions de tache sont protégées de l'hybridation et/ou de la ligature,
facultativement dans lequel les molécules d'oligonucléotides dans la ou les région(s) de tache sont protégées de l'hybridation et/ou de la ligature par une photorésine recouvrant les molécules d'oligonucléotides,
facultativement dans lequel la photorésine dans les régions de tache irradiées est éliminée et la photorésine dans les régions de tache masquées ou non irradiées n'est pas éliminée.

6. Procédé selon l'une quelconque des revendications 1 à 5, dans lequel, pendant et après l'irradiation à l'étape c), les molécules d'oligonucléotides dans les une ou plusieurs autres régions de tache sont protégées de l'hybridation et/ou de la ligature,
facultativement dans lequel les molécules d'oligonucléotides dans la ou les région(s) de tache sont protégées de l'hybridation et/ou de la ligature par une photorésine recouvrant les molécules d'oligonucléotides,
facultativement dans lequel la photorésine dans les régions de tache irradiées est éliminée et la photorésine dans les régions de tache masquées ou non irradiées n'est pas éliminée.

7. Procédé selon l'une quelconque des revendications 1 à 6, dans lequel
i) les molécules d'oligonucléotides dans la ou les région(s) de tache sont protégées de l'hybridation et/ou de la ligature par un groupe protecteur de chaque molécule d'oligonucléotide,
facultativement dans lequel le groupe protecteur est un groupe protecteur photoclivable, facultativement dans lequel les groupes protecteurs photoclivables dans les régions irradiées à l'étape c) sont clivés et les groupes protecteurs photoclivables dans les régions masquées ou non irradiées à l'étape c) ne sont pas clivés, ou
ii) les molécules d'oligonucléotides dans la ou les région(s) de tache sont protégées de l'hybridation et/ou de la ligature par un polymère se liant aux molécules d'oligonucléotides,
facultativement dans lequel le polymère est un polymère photoclivable, facultativement dans lequel le polymère se lie aux molécules d'oligonucléotides d'une manière non spécifique à la séquence,
facultativement, dans lequel les polymères photoclivables dans la première ou les plusieurs taches irradiées des régions de tache sont clivées et les polymères photoclivables dans une ou plusieurs autres régions de tache masquées ou non irradiées ne sont pas clivées.

8. Procédé selon l'une quelconque des revendications 1 à 7, comprenant l'irradiation du substrat dans de multiples cycles, chaque cycle pour irradier une ou plusieurs régions de tache qui sont différentes des une ou plusieurs régions de tache irradiées dans un autre cycle et la fixation d'un oligonucléotide d'une longueur de l'au moins quatre nucléotides à la molécule d'oligonucléotide immobilisée dans la ou les région(s) de tache irradiée(s) pour le cycle.

9. Procédé selon l'une quelconque des revendications 1 à 8, dans lequel l'irradiation à l'étape c) est réalisée en utilisant un photomasque, dans lequel la première ou plusieurs des régions de tache sont démasquées et les une ou plusieurs autres régions de tache sont masquées.

10. Procédé selon la revendication 9, comprenant la translation du photomasque utilisé à l'étape d'irradiation en c) d'une première position à une deuxième position par rapport au substrat, chaque position pour un cycle d'irradiation du substrat et de fixation d'un oligonucléotide.

11. Procédé selon l'une quelconque des revendications 1 à 10, dans lequel les molécules d'oligonucléotides dans la première ou plusieurs des régions de tache, les une ou plusieurs autres régions de tache, et la région limite sont identiques en séquence avant l'irradiation à l'étape a).

12. Procédé selon l'une quelconque des revendications 1 à 11, dans lequel la pluralité de régions de tache ont, en moyenne, un diamètre non supérieur à 55 micromètres,
facultativement, dans lequel la pluralité de régions de tache ont, en moyenne, un diamètre non supérieur à 5 micromètres.

13. Procédé selon l'une quelconque des revendications 1 à 12, dans lequel la région limite comprend la région sur le substrat entourant la pluralité de régions de tache et entre celles-ci.

14. Procédé selon l'une quelconque des revendications 1 à 13, dans lequel les molécules d'oligonucléotides sur le substrat avant l'étape d'irradiation ont une longueur entre environ 4 et environ 50 nucléotides.

15. Procédé selon l'une quelconque des revendications 1 à 14, dans lequel les molécules d'oligonucléotides sont appliquées au substrat en tant que tapis avant l'irradiation à l'étape a).
